(19) Europäisches Patentamt
European Patent Office
Office européen des brevets

(11) Veröffentlichungsnummer: **0 563 734 A1**

# (12) EUROPÄISCHE PATENTANMELDUNG

(21) Anmeldenummer: **93104662.7**

(22) Anmeldetag: **22.03.93**

(51) Int. Cl.[5]: **C07D 498/06**, A61K 31/535, //(C07D498/06,265:00,221:00)

(30) Priorität: **02.04.92 DE 4210941**

(43) Veröffentlichungstag der Anmeldung: **06.10.93 Patentblatt 93/40**

(84) Benannte Vertragsstaaten: **AT BE CH DE DK ES FR GB GR IE IT LI LU MC NL PT SE**

(71) Anmelder: **BAYER AG**
**D-51368 Leverkusen(DE)**

(72) Erfinder: **Schneider, Stephan, Dr.**
**116 Acorn Road**
**Madison, CT 06443(US)**
Erfinder: **Ruppelt, Martin, dr.**
**Lützenberger Strasse 351**
**W-5600 Wuppertal 1(DE)**
Erfinder: **Schriewer, Michael, Dr.**
**Am Thelen Siefen 1a**
**W-5068 Odenthal(DE)**
Erfinder: **Schulze, Thomas J., Dr.**
**Jakob-Böhme-Strasse 11**
**W-5000 Köln 80(DE)**
Erfinder: **Neumann, Rainer, Dr.**
**Albert-Einstein-Strasse 27**
**W-5024 Pulheim 2(DE)**

(54) **Neue 9-Fluor-7-oxo-7H-pyrido 1,2-3-de 1,4 benzoxazin-6-carbonsäuren und -ester und ihre Verwendung als antivirale Mittel.**

(57) Die vorliegende Erfindung betrifft neue 9-Fluor-7-oxo-7H-pyrido[1,2,3-d,e][1,4]benzoxacin-6-carbonsäuren und -ester der allgemeinen Formel (I), Verfahren zu ihrer Herstellung sowie ihre Verwendung als Arzneimittel, insbesondere als antivirale Arzneimittel.

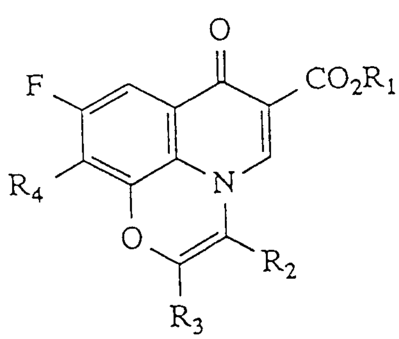

(I),

EP 0 563 734 A1

Die vorliegende Erfindung betrifft neue 9-Fluor-7-oxo-7H-pyrido[1,2,3-de][1,4]-benzoxazin-6-carbonsäuren und -ester, Verfahren zu ihrer Herstellung sowie ihre Verwendung als Arzneimittel, insbesondere als antivirale Arzneimittel.

Aus der EP-A-253 235 sind bereits 1,8-verbrückte 4-Chinoloncarbonsäuren mit einer antibakteriellen Wirkung bekannt.

Die vorliegende Erfindung betrifft neue 9-Fluor-7-oxo-7H-pyrido[1,2,3-de][1,4]-benzoxazin-6-carbonsäuren und -ester der allgemeinen Formel (I)

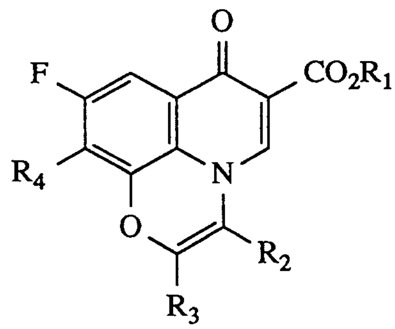

(I),

in welcher

| | |
|---|---|
| $R^1$ | für Wasserstoff oder geradkettiges oder verzweigtes Alkyl mit bis zu 8 Kohlenstoffatomen steht, |
| $R^2$ | für Wasserstoff, Methyl, Formyl, Benzyl, p-Cl-Benzyl, Carboxy oder für die $-CONH_2$-Gruppe steht, oder<br>für geradkettiges oder verzweigtes Alkenyl oder Alkoxycarbonyl mit jeweils bis zu 6 Kohlenstoffatomen steht, oder<br>für geradkettiges oder verzweigtes, durch Carboxy, Benzyloxy, Imidazolyl oder durch geradkettiges oder verzweigtes Alkoxycarbonyl mit bis zu 4 Kohlenstoffatomen substituiertes Alkyl mit bis zu 6 Kohlenstoffatomen steht, oder<br>für Cycloalkyl mit 3 bis 8 Kohlenstoffatomen steht, |
| $R^3$ | für Wasserstoff,<br>für Carboxy oder<br>für Cycloalkyl mit 3 bis 8 Kohlenstoffatomen steht, oder<br>für geradkettiges oder verzweigtes Alkoxycarbonyl mit bis zu 8 Kohlenstoffatomen, oder<br>für geradkettiges oder verzweigtes, durch Carboxy, Phenoxy oder durch geradkettiges oder verzweigtes Alkoxycarbonyl mit bis zu 6 Kohlenstoffatomen substituiertes Alkyl mit bis zu 10 Kohlenstoffatomen steht, oder<br>für Biphenyl oder für einen Rest der Formel |

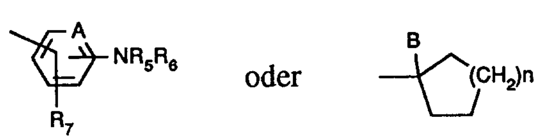

| | |
|---|---|
| | steht,<br>worin |
| A | ein Stickstoffatom oder die -CH-Gruppe bedeutet, |
| $R^5$ und $R^6$ | gleich oder verschieden sind und Wasserstoff oder geradkettiges oder verzweigtes Alkyl oder Acyl mit jeweils bis zu 6 Kohlenstoffatomen bedeuten, |
| $R^7$ | Wasserstoff, Halogen, Hydroxy oder geradkettiges oder verzweigtes Alkoxy oder Alkyl mit jeweils bis zu 6 Kohlenstoffatomen bedeutet, |
| n | eine Zahl 1,2 oder 3 bedeutet,<br>und |
| B | geradkettiges oder verzweigtes Alkyl mit bis zu 6 Kohlenstoffatomen oder Wasserstoff |

bedeutet,

$R^4$ für über N-gebundenes Imidazolyl oder 1,4-Diazacycloheptanyl steht, oder für einen Rest der Formel

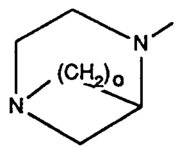 , 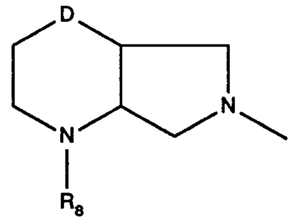 , 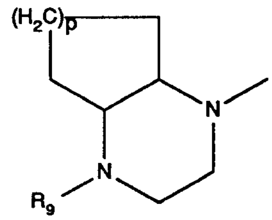 ,

steht,

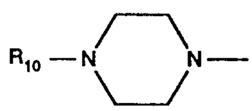 oder 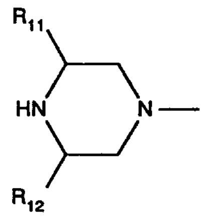

worin

o eine Zahl 2 oder 3 bedeutet,

p eine Zahl 1 oder 2 bedeutet,

D die $-CH_2$-Gruppe oder ein Sauerstoffatom bedeutet,

$R^8$ und $R^9$ gleich oder verschieden sind und Wasserstoff, Methyl oder Ethyl bedeuten,

$R^{10}$ Cycloalkyl mit 3 bis 8 Kohlenstoffatomen oder geradkettiges oder verzweigtes Perfluoralkyl mit bis zu 6 Kohlenstoffatomen bedeutet, oder Phenyl, Pyridyl oder Pyrimidyl bedeutet, die gegebenenfalls durch Halogen oder geradkettiges oder verzweigtes Alkyl oder Alkoxy mit jeweils bis zu 6 Kohlenstoffatomen substituiert sind,

$R^{11}$ Wasserstoff oder Methyl bedeutet,

$R^{12}$ geradkettiges oder verzweigtes, durch Hydroxy, Benzyloxy, Phenoxy oder Morpholino substituiertes Alkyl mit bis zu 6 Kohlenstoffatomen bedeutet,

und im Fall, daß $R^3$ nicht Wasserstoff bedeutet, wenn $R^2$ für Wasserstoff oder Methyl steht,

$R^4$ außerdem für Morpholino oder für einen Rest der Formel

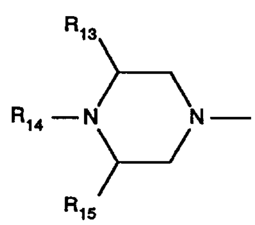

steht,

worin

$R^{13}$, $R^{14}$ und $R^{15}$ gleich oder verschieden sind und Wasserstoff, Hydroxyethyl, Methyl oder Ethyl bedeuten

und deren Hydrate und Salze, gegebenenfalls in einer isomeren Form.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Alkali-, Erdalkali, Silber-und Guanidiniumsalze der erfindungsgemäßen Verbindungen sein.

Bevorzugt sind Verbindungen der allgemeinen Formel (I),

in welcher

$R^1$ für Wasserstoff, oder
für geradkettiges oder verzweigtes Alkyl mit bis zu 6 Kohlenstoffatomen steht,

$R^2$ für Wasserstoff, Formyl, Methyl, Benzyl, p-Cl-Benzyl, Carboxy oder für die $-CONH_2$-Gruppe steht, oder
für geradkettiges oder verzweigtes Alkenyl oder Alkoxycarbonyl mit jeweils bis zu 4 Kohlenstoffatomen steht, oder
für geradkettiges oder verzweigtes, durch Carboxy, Benzyloxy, Imidazolyl, Methoxycarbonyl, Ethoxycarbonyl oder Propoxycarbonyl substituiertes Alkyl mit bis zu 4 Kohlenstoffatomen steht, oder
für Cyclopropyl, Cyclobutyl, Cyclopentyl oder Cyclohexyl steht,

$R^3$ für Wasserstoff,
für Carboxy, oder
für Cyclopropyl, Cyclobutyl, Cyclopentyl oder Cyclohexyl steht, oder
für geradkettiges oder verzweigtes Alkoxycarbonyl mit bis zu 6 Kohlenstoffatomen steht, oder
für geradkettiges oder verzweigtes, durch Carboxy, Phenoxy oder durch geradkettiges oder verzweigtes Alkoxycarbonyl mit bis zu 4 Kohlenstoffatomen substituiertes Alkyl mit bis zu 8 Kohlenstoffatomen steht, oder
für Biphenyl oder für einen Rest der Formel

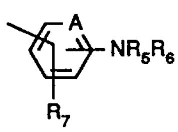 oder 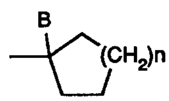

steht,
worin

A ein Stickstoffatom oder die -CH-Gruppe bedeutet,

$R^5$ und $R^6$ gleich oder verschieden sind und Wasserstoff oder geradkettiges oder verzweigtes Alkyl oder Acyl mit jeweils bis zu 4 Kohlenstoffatomen bedeuten,

$R^7$ Wasserstoff, Fluor, Chlor, Hydroxy oder geradkettiges oder verzweigtes Alkoxy oder Alkyl mit jeweils bis zu 4 Kohlenstoffatomen bedeutet,

n eine Zahl 1 oder 2 bedeutet,
und

B geradkettiges oder verzweigtes Alkyl mit bis zu 4 Kohlenstoffatomen oder Wasserstoff bedeutet,

$R^4$ für über N-gebundenes Imidazolyl oder 1,4-Diazacycloheptanyl steht, oder
für einen Rest der Formel

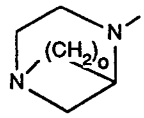 , 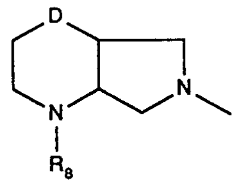 , 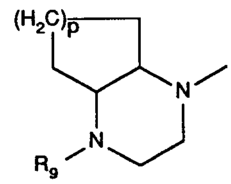 ,

steht,

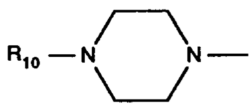 oder 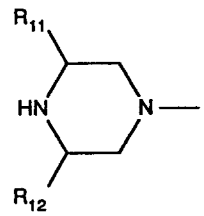

worin

o eine Zahl 2 oder 3 bedeutet,

p eine Zahl 1 oder 2 bedeutet,

D die $-CH_2$-Gruppe oder ein Sauerstoffatom bedeutet,

$R^8$ und $R^9$ gleich oder verschieden sind und Wasserstoff, Methyl oder Ethyl bedeuten,

$R^{10}$ Cyclopropyl, Cyclopentyl oder Cyclohexyl bedeutet, oder
geradkettiges oder verzweigtes Perfluoralkyl mit bis zu 4 Kohlenstoffatomen bedeutet, oder
Phenyl, Pyridyl oder Pyrimidyl bedeutet, die gegebenenfalls durch Fluor, Chlor oder geradkettiges oder verzweigtes Alkyl oder Alkoxy mit jeweils bis zu 4 Kohlenstoffatomen substituiert sind,

$R^{11}$ Wasserstoff oder Methyl bedeutet,

$R^{12}$ geradkettiges oder verzweigtes, durch Hydroxy, Benzyloxy, Phenoxy oder Morpholino substituierte, Alkyl mit bis zu 4 Kohlenstoffatomen bedeutet,

und im Fall, daß $R^3$ nicht Wasserstoff bedeutet, wenn $R^2$ für Wasserstoff oder Methyl steht,

$R^4$ außerdem für Morpholino oder für einen Rest der Formel

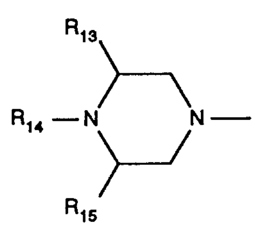

steht,
worin

$R^{13}$, $R^{14}$ und $R^{15}$ gleich oder verschieden sind und Wasserstoff, Hydroxyethyl, Methyl oder Ethyl bedeuten

und deren Hydrate und Salze, gegebenenfalls in einer isomeren Form.

Besonders bevorzugt sind Verbindungen der allgemeinen Formel (I),
in welcher

$R^1$ für Wasserstoff oder für geradkettiges oder verzweigtes Alkyl mit bis zu 4 Kohlenstoffatomen steht,

$R^2$ für Wasserstoff, Formyl, Methyl, Benzyl, p-Cl-Benzyl, Carboxy oder für die $-CONH_2$-Gruppe steht,
für Vinyl, Allyl, Methoxycarbonyl oder Ethoxycarbonyl steht,
für geradkettiges oder verzweigtes, durch Carboxy, Benzyloxy, Imidazolyl, Methoxycarbonyl oder Ethoxycarbonyl substituiertes Alkyl mit bis zu 3 Kohlenstoffatomen steht, oder für Cyclopropyl oder Cyclopentyl steht, oder

$R^3$ für Wasserstoff, oder
für Carboxy, oder
für Cyclopropyl oder Cyclopentyl steht, oder
für geradkettiges oder verzweigtes Alkoxycarbonyl mit bis zu 4 Kohlenstoffatomen steht, oder
für geradkettiges oder verzweigtes, durch Carboxy, Phenoxy oder durch geradkettiges oder verzweigtes Alkoxycarbonyl mit bis zu 3 Kohlenstoffatomen substituiertes Alkyl mit bis zu 6 Kohlenstoffatomen steht, oder
für einen Rest der Formel

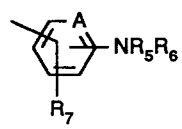

steht,
worin

A ein Stickstoffatom oder die -CH-Gruppe bedeutet,

$R^5$ und $R^6$ gleich oder verschieden sind und Wasserstoff, Methyl, Ethyl oder Acetyl bedeuten,

$R^7$ Wasserstoff, Fluor, Chlor, Methyl, Ethyl, Methoxy oder Ethoxy bedeutet,

$R^4$ für über N-gebundenes Imidazolyl oder 1,4-Diazacycloheptanyl steht, oder
für einen Rest der Formel

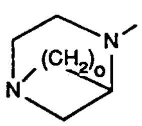 , 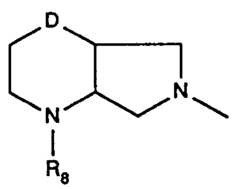 , 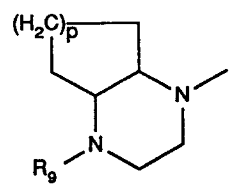 ,

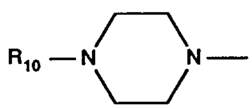 oder 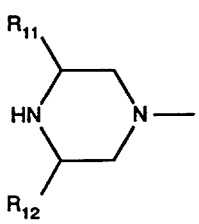

steht,

worin

o die Zahl 2 bedeutet,

p eine Zahl 1 oder 2 bedeutet,

D die $-CH_2$-Gruppe oder ein Sauerstoffatom bedeutet,

$R^8$ und $R^9$ gleich oder verschieden sind und Wasserstoff, Methyl oder Ethyl bedeuten,

$R^{10}$ Cyclopropyl oder geradkettiges oder verzweigtes Perfluoralkyl mit bis zu 3 Kohlenstoffatomen oder Phenyl, Pyridyl oder Pyrimidyl bedeutet, die gegebenenfalls durch Fluor, Chlor oder geradkettiges oder verzweigtes Alkyl oder Alkoxy mit jeweils bis zu 3 Kohlenstoffatomen substituiert sind,

$R^{11}$ Wasserstoff oder Methyl bedeutet,

$R^{12}$ geradkettiges oder verzweigtes, durch Hydroxy, Benzyloxy, Phenoxy oder Morpholin substituiertes Alkyl mit bis zu 3 Kohlenstoffatomen bedeutet,

und im Fall, daß $R^3$ nicht Wasserstoff bedeutet, wenn $R^2$ für Wasserstoff oder Methyl steht,

$R^4$ außerdem für Morpholino oder für einen Rest der Formel

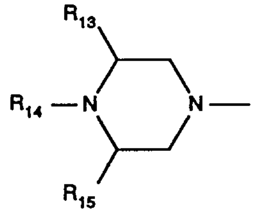

steht,
worin

$R^{13}$, $R^{14}$ und $R^{15}$ gleich oder verschieden sind und Wasserstoff, Hydroxyethyl, Methyl oder Ethyl bedeuten

und deren Hydrate und Salze, gegebenenfalls in einer isomeren Form.

Außerdem wurden Verfahren zur Herstellung der erfindungsgemäßen Verbindungen der allgemeinen Formel (I) gefunden, dadurch gekennzeichnet,
daß man

[A] Verbindungen der allgemeinen Formel (II)

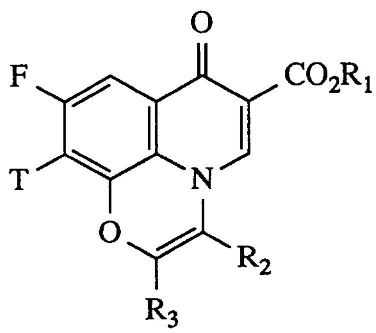

(II),

in welcher

$R^2$ und $R^3$ die oben angegebene Bedeutung haben,

$R^1$ die oben angegebene Bedeutung hat, aber vorzugsweise für Wasserstoff steht,

und

T für Halogen, vorzugsweise für Fluor steht,

mit Verbindungen der allgemeinen Formel (III)

$R^4$-H (III),

in welcher

$R^4$ die oben angegebene Bedeutung hat,

in inerten Lösemitteln, in Anwesenheit einer Base unter Schutzgasatmosphäre umsetzt,
oder
Aldehyde der allgemeinen Formel (IV)

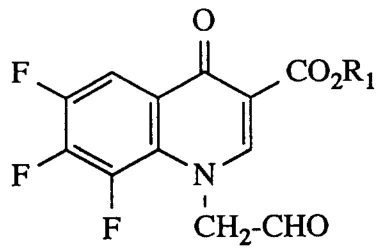

(IV),

in welcher

$R^1$ die oben angegebene Bedeutung hat,

zunächst mit Verbindungen der allgemeinen Formel (III) oder (IIIa), vorzugsweise mit (IIIa)

$R^4$-H (III),

W-H (IIIa),

in welcher

$R^4$ die oben angegebene Bedeutung hat,

W die oben angegebene Bedeutung von $R^4$ hat, wobei eine der cyclischen Aminfunktionen durch eine Schutzgruppe, vorzugsweise tert.Butoxycarbonyl oder Ethoxycarbonyl, geschützt ist,

in inerten Losemitteln, in Anwesenheit einer Base unter gleichzeitiger Cyclisierung umsetzt, die Schutzgruppe nach üblicher Methode abspaltet

oder im Fall, daß $R^2$, wie oben definiert, für substituiertes Alkyl steht,

[B] Verbindungen der allgemeinen Formel (V)

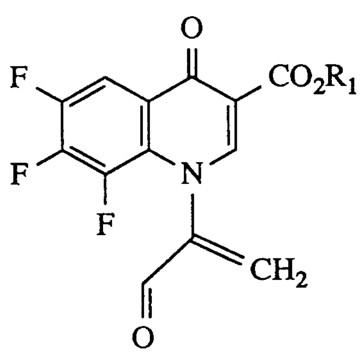

(V),

in welcher

$R^1$ die oben angegebene Bedeutung hat,

mit Verbindungen der allgemeinen Formel (VI)

$R^{17}$-H (VI),

in welcher

$R^{17}$ für das oben unter $R^2$ aufgeführte substituierte Alkyl steht, das um ein Kohlenstoffatom verkürzt ist,

in einem der oben aufgeführten Lösemitteln, gegebenenfalls in Anwesenheit einer Base unter gleichzeitiger Cyclisierung umsetzt, und in einem letzten Schritt den Substituenten $R^4$, wie oben beschrieben, einführt,

und im Fall, daß $R^3 \neq$ H, derivatisiert,

und anschließend je nach gewünschter Bedeutung für $R^1$ entweder eine Veresterung oder Verseifung nach üblicher Methode durchführt.

Das erfindungsgemäße Verfahren kann durch folgendes Formelschema beispielhaft erläutert werden:

[A]

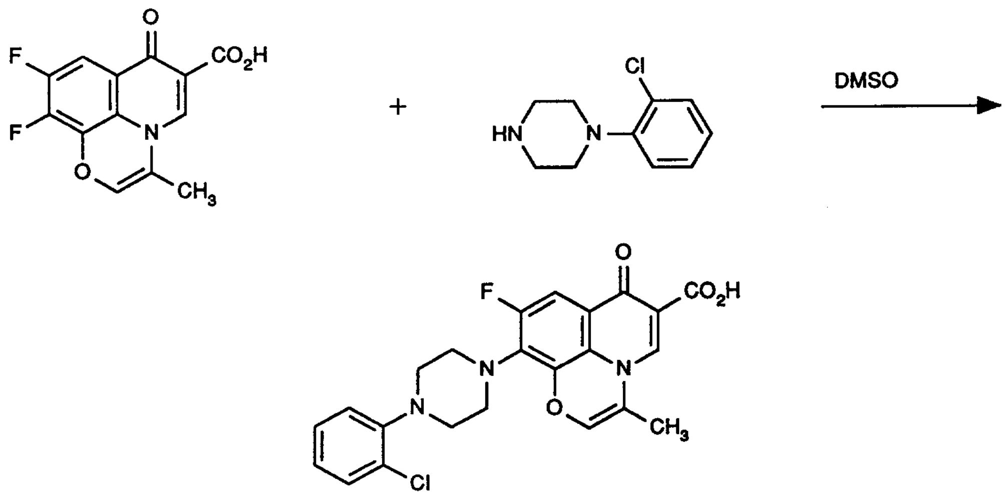


[B]

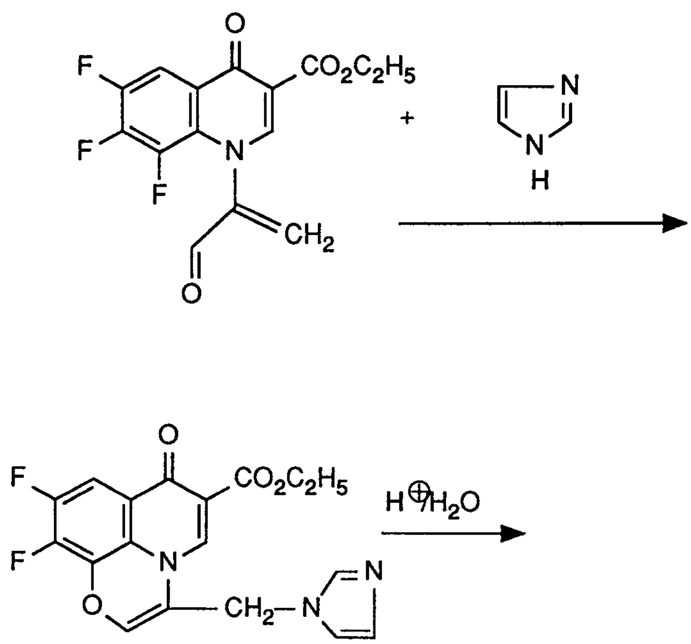

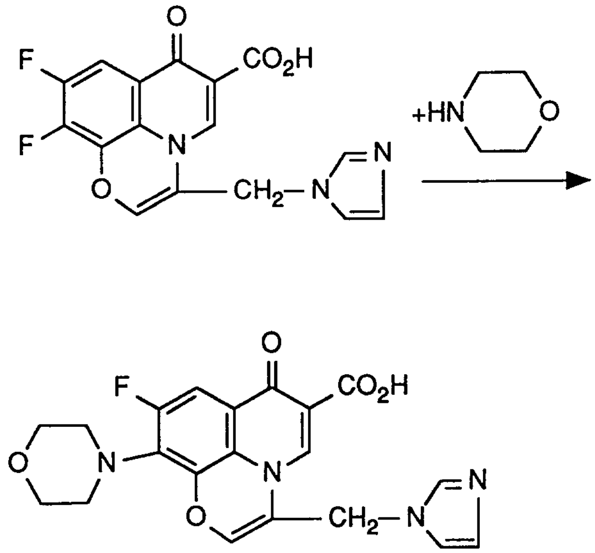

Schutzgruppen im Rahmen der Erfindung sind die aus der Peptid-Chemie bekannten Gruppen wie beispielsweise Benzyloxycarbonyl, Methoxycarbonyl, Ethoxycarbonyl, Propoxycarbonyl, Isopropoxycarbonyl, Butoxycarbonyl oder Isobutoxycarbonyl. Bevorzugt sind Methoxy- und Ethoxycarbonyl.

Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether z.B. Diethylether, Glykolmono-oder -dimethylether, Dioxan oder Tetrahydroluran, oder Kohlenwasserstoffe wie Benzol, Toluol, Xylol, Cyclohexan oder Erdölfraktionen, oder Halogenkohlenwasserstoffe wie Methylenchlorid, Chloroform, Tetrachlorkohlenstoff, oder Dimethylsulfoxid, N,N-Dimethylformamid, Hexamethylphosphorsäuretriamid, Sulfolan, Essigester, Pyridin, Triethylamin, N-Methylpyrrolidin, Anisol oder Picolin. Ebenso ist es möglich, Gemische der genannten Lösemittel zu verwenden. Bevorzugt sind Dimethylsulfoxid und N,N-Dimethylformamid.

Als Basen eignen sich die üblichen basischen Verbindungen. Hierzu gehören beispielsweise Alkali- oder Erdalkalihydroxide oder -carbonate, Pyndin, Triethylamin, Düsopropylethylamin oder N-Methylpiperidin, oder bicyctische Amidine wie 1,5-Diazabicyclo[3,4,0]-nonene-5 (DBN), 1,5-Diazabicyclo[3,4,0]undecene-5 (DBU) oder DABCO. Bevorzugt sind Triethylamin, Düsopropylethylamin, DABCO und Kaliumcarbonat.

Die Basen werden im allgemeinen in einer Menge von 1 bis 3 mol, bevorzugt von 1 bis 1,5 mol, bezogen auf 1 mol der entsprechenden Carbonsäure, eingesetzt.

Das Verfahren wird im allgemeinen in einem Temperaturbereich von +0°C bis +150°C, bevorzugt von +0°C bis +120°C, durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Als Lösemittel eignen sich für die Verseitung Wasser oder Wasser in Kombination mit einem üblichen organischen Lösermittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet.

Die Verseitung erfolgt mit Säuren wie beispielsweise Essigsäure, Chlorwasserstoffsäure, Bromwasserstoffsäure, Methansulfonäsure, Schwefelsäure oder Perchlorsäure.

Die Verseifung wird im allgemeinen in einem Temperaturbereich von 0°C bis +130°C, bevorzugt von +20°C bis +110°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Säure im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Die Verseifung von tert.Butylestern erfolgt im allgemeinen mit Säuren, wie beispielsweise Salzsäure oder Trifluoressigsäure, in Anwesenheit eines der oben angegebenen Lösemittel und/oder Wasser oder deren Gemische, vorzugsweise mit Dioxan oder Tetrahydrofuran.

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dann der entsprechende Alkohol auch als Lösemittel eingesetzt.

Als Katalysatoren können anorganische Säuren, wie bespielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator, bezogen auf 1 mol Reaktionspartner, ein.

Die Abspaltung der Aminoschutzgruppen erfolgt nach literaturbekannten Methoden [vgl. Houben-Weyl, "Methoden der organischen Chemie", Synthese von Peptiden II, 4. Auflage, Bd. 15/1,15/2, Georg Thieme Verlag], bevorzugt mit Trifluoressigsäure in Anisol.

Die Verbindungen der allgemeinen Formel (II) sind als konkrete Stoffvertreter teilweise bekannt [vgl. hierzu EP 253 235 A1] oder neu und können beispielsweise hergestellt werden, indem man Verbindungen der allgemeinen Formel (VII)

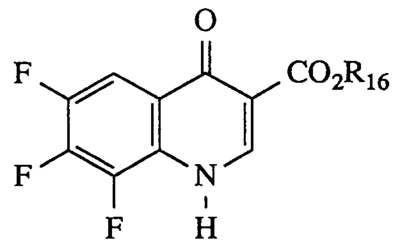

(VII),

in welcher

$R^{16}$ für einen $C_1$-$C_4$-Alkylrest steht,

mit einer $\alpha$-Halogencarbonylverbindung der allgemeinen Formel (VIII)

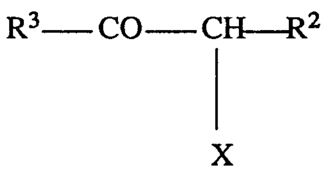

(VIII),

in welcher

$R^2$ und $R^3$ die oben angegebene Bedeutung haben

und

X für Halogen, vorzugsweise für Brom steht,

in einem der oben aufgeführten Lösemitteln und Basen, vorzugsweise Dimethylformamid und Kaliumcarbonat, umsetzt,

oder

indem man Verbindungen der allgemeinen Formel (IX)

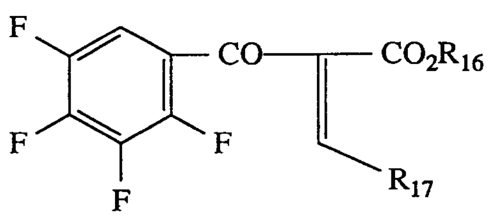

(IX),

in welcher

$R^{16}$ die oben angegebene Bedeutung hat und

$R^{17}$ für $C_1$-$C_4$-Alkoxy steht,

mit Verbindungen der allgemeinen Formel (X)

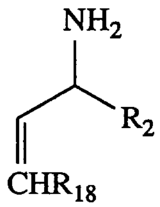

(X),

in welcher

$R^2$ die oben angegebene Bedeutung hat und

$R^{18}$ für Wasserstoff oder $C_1$-$C_4$-Alkyl steht,

in einem der oben aufgeführten Lösemitteln, vorzugsweise Ethanol, und gegebenenfalls in Anwesenheit einer dort ebenfalls aufgeführten Aminbase, umsetzt und anschließend mit Dimethoxyethan und Natriumhydrid in die Verbindungen der allgemeinen Formel (XI)

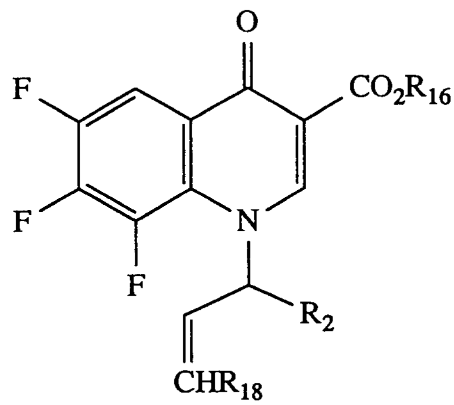

(XI),

in welcher

$R^2$, $R^{16}$ und $R^{18}$ die oben angegebene Bedeutung haben,

überführt,

anschließend durch Ozonolyse nach üblichen Methoden die Doppelbindung zur Carbonylfunktion spaltet, und in einem letzten Schritt die Cyclisierung wie oben beschrieben durchführt, und im Fall der oben unter $R^2$ und $R^3$ aufgeführten weiteren Substituenten diese nach üblichen Methoden variiert.

Die Umsetzung mit den Verbindungen der allgemeinen Formel (X) erfolgt in einem Temperaturbereich von -20 °C bis +30 °C, vorzugsweise von 0 °C bis Raumtemperatur, und Normaldruck.

Die Ozonolyse wird im allgemeinen bei -78 °C in einem Lösemittelgemisch Methanol/Methylenchlorid und unter Schutzgasatmosphäre durchgeführt.

Die Verbindungen der allgemeinen Formeln (IX) und (X) sind teilweise bekannt, oder können nach üblichen Methoden hergestellt werden.

Die Verbindungen der allgemeinen Formel (XI) sind größtenteils neu und können nach dem oben beschriebenen Verfahren hergestellt werden.

Die Verbindungen der allgemeinen Formeln (VII) und (VIII) sind an sich bekannt [vgl. hierzu EP 253 235 Al] oder können nach üblicher Methode hergestellt werden.

Ebenso sind die Verbindungen der allgemeinen Formeln (III) und (IIIa) bekannt, teilweise käuflich oder nach üblichen Methoden herstellbar.

Die Aldehyde der allgemeinen Formel (IV) sind an sich bekannt oder können nach publizierten Methoden hergestellt werden [vgl. EP 253 235 A1].

Die Verbindungen der allgemeinen Formel (V) sind neu und können hergestellt werden, indem man Verbindungen der allgemeinen Formel (XII)

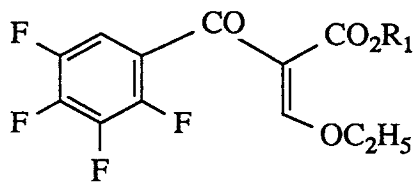

(XII),

in welcher

$R^1$ die oben angegebene Bedeutung hat,

durch eine 2-Stufen-Umsetzung mit 3-Amino-4-berzyloxy-1-buten der Formel (XIII)

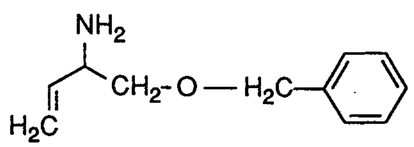

(XIII)

in einem der oben aufgeführten Lösemittel, vorzugsweise Ethanol, und anschließend mit Natriumhydrid in wasserfreiem Dimethoxyethan in die Verbindung der Formel (XIV)

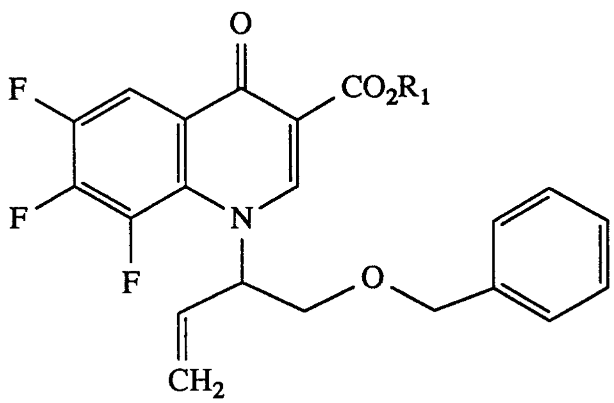

(XIV),

in welcher

$R^1$ die oben angegebene Bedeutung hat,

überführt,

und anschließend eine Ozonolyse durchführt.

Die Ozonolyse wird im allgemeinen bei -78°C in einem Losemittelgemisch Methanol/Methylenchlorid und unter Schutzgasatmosphäre durchgeführt.

Die Verbindung der Formel (XIV) ist neu und kann nach den oben aufgeführten Verfahren hergestellt werden.

Die Verbindungen der Formeln (XII) und (XIII) sind bekannt.

Die erfindungsgemäßen Verbindungen der Formel (I) zeigen ein weiteres nicht vorhersehbares, wertvolles pharmakologisches Wirkspektrum. Sie sind überraschenderweise geeignet zur Behandlung von virusinduzierten Erkrankungen.

Ebenso können die Zwischenprodukte der allgemeinen Formel (II) dieses Wirkspektrum aufweisen.

Der Nachweis wurde in mit Hepatitis B Virus DNA transfizierten Hepatomzellen (HEP G2.2.15) geführt. Die Ergebnisse der unten aufgeführten Beispiele wurden zu dem in der folgenden Literaturangabe [Sells, M.A.; Chen, M.L., Acs, G., Proc. Natl. Acad. Sci. USA S. 1005 - 1009, Vol. 84 (1987] beschriebenen HBV-Testsystem ermittelt:

Transfizierte Hepatomzellen (HEP G2.2.15) wurden mit verschiedenen Konzentrationen der jeweiligen Verbindung inkubiert. Durch Behandlung der transfizierten Hepatomzellinie HEP G2.2.15 mit den erfindungsgemäßen Verbindungen konnte die Reduktion der virusspezifischen HBV-DNA im Überstand sowie eine Reduktion des HBsAg Spiegels gezeigt werden. Es wurde weiter gefunden, daß die erfindungsgemäßen Verbindungen zu einer Reduktion der replikativen Intermediate der Hepatitisviren führen. Im Überstand der Zellkulturen wurde nach PEG Fällung der Gehalt an HBV-DNA bestimmt. Dies geschah unter Verwendung einer nicht radioaktiv markierten HBV genomischen DNA Probe [Pauly, P., 1982, Ph. D. Thesis, University of Göttingen, F.R.G.; Köchel et al., 1990, EMBL, Accession-Nr. X 51790]. Gleichzeitig erfolgte der Nachweis der Beeinflussung der HBsAg Bildung mittels eines kommerziell erhältlichen ELISA Tests. Die angegebenen $IC_{50}$-Werte beziehen sich auf die Substratkonzentrationen, die unter den oben aufgeführten Testbedingungen eine 50%ige Hemmung der HBV-DNA Konzentration im Überstand bewirken.

Tabelle A

| Bsp.Nr. | $IC_{50}$ ($\mu$M) | SI |
|---|---|---|
| I | 0,5 | ~ 100 |
| VIII | 0,5 | > 100 |
| 9 | 0,1 | ~ 20 |

Gleichzeitig erfolgte die Bestimmung des möglicherweise vorhandenen cytotoxischen Einflusses der erfindungsgemäßen Verbindungen mittels Kristallviolettfärbung bzw. über den Einbau von radioaktiv markiertem Thymidin in die zelluläre DNA.

Die Wirkung der erfindungsgemäßen Verbindungen auf andere Zellen wurde durch Inkubation von HEL-, MEF-Zellen getestet. Es zeigte sich keine Beeinflussung der Vitalität dieser Zellen bis zu einer Konzentration von 250 $\mu$M.

Als Indikationsgebiete für die erfindungsgemäß verwendbaren Verbindungen können beispielsweise genannt werden:

Die Behandlung von akuten und chronischen Virusinfektionen, die zu einer Hepatitis führen können, beispielsweise die Infektionen mit Hepatitisviren; ferner Virusinfektionen mit Herpesviren Typ 1, 2, Zytomegaloviren, Varizella-Zoster-Viren und HIV.

Besonders bevorzugt ist die Behandlung von chronischen Hepatitis-B Virusinfektionen und die Behandlung von akuter Hepatitis-B Virusinfektion.

Zur vorliegenden Erfindung gehören pharmazeutische Zubereitungen, die neben nicht-toxischen, inerten pharmazeutisch geeigneten Trägerstoffen eine oder mehrere Verbindungen der Formeln (I) oder (II) enthalten oder die aus einem oder mehreren Wirkstoffen der Formeln (I) und (II) bestehen, sowie Verfahren zur Herstellung dieser Zubereitungen.

Die Wirkstoffe der Formeln (I) und (II) sollten in den oben aufgeführten pharmazeutischen Zubereitungen in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den Verbindungen der Formeln (I) und (II) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Ausgangsverbindungen

Beispiel I

9,10-Difluor-2-ethoxycarbonyl-7-oxo-7H-pyrido[1,2,3-de][1,4]benzoxazin-6-carbonsäureethylester

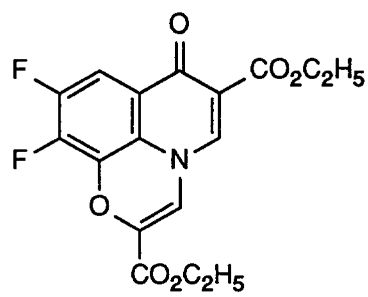

Methode [A]

Die Verbindung wird entweder nach den in EP-A-253 235 publizierten Verfahren hergestellt oder über die im folgenden aufgeführten Zwischenstufen a) und b).

Methode [B]

a) 6,7,8-Trifluoro-1-(2-ethoxycarbonyl-2-propenyl)-4-oxo-1,4-dihydrochinolin-3-carbonsäureethylester

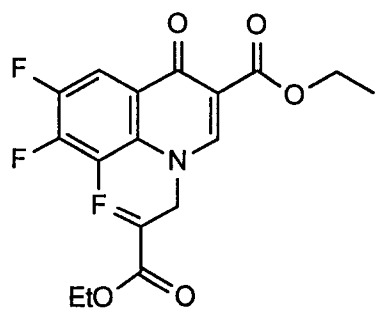

Unter Argon erhitzt man 7 g (25,9 mmol) 6,7,8-Trifluor-4-hydroxy-3-chinolincarbonsäureethylester und 4,3 g (31,1 mmol) $K_2CO_3$ in 60 ml DMF 30 Minuten auf 80 °C. Man entfernt das Heizbad und addiert 5 g (25,9 mmol) 2-(Brommethyl)-acrylsäureethylester in 20 ml DMF. Anschließend wird 4 h bei 80 °C gerührt. Nach dem Abkühlen wird der Feststoff abfiltriert und die Lösung eingeengt. Der Rückstand wird mit 1N HCl sauer gestellt, mehrfach mit $CH_2Cl_2$ extrahiert, über $MgSO_4$ getrocknet und eingeengt. Dieses Rohprodukt wird mit dem abfiltrierten Feststoff vereinigt und chromatographisch an Kieselgel ($CH_2Cl$:MeOH:$NH_{3(aq)}$ = 100:5:2) gereinigt. Nach Umkristallisation aus Essigester erhält man 5,54 g (56 % der Theorie) der Titelverbindung.
Schmelzpunkt: 188 °C.

b) 6,7,8-Trifluoro-1-(2-ethoxycarbonyl-2-oxo-ethyl)-4-oxo-1,4-dihydrochinolin-3-carbonsäureethylester

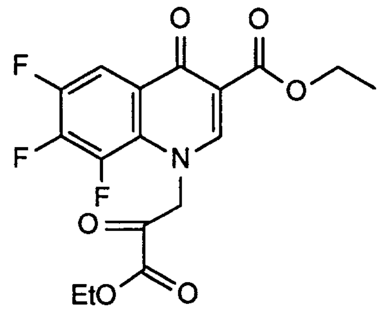

1 g (2,61 mmol) der Verbindung aus a) werden in 150 mi eines Gemisches (1:1) aus $CH_2Cl_2$ und MeOH gelöst und auf -78 °C gekühlt. Anschließend wird Ozon bis zu Blaufärbung eingeleitet. Überschüssiges Ozon wird durch Sauerstoffgas vertrieben. Man vernetzt mit 5 ml Dimethylsulfid, läßt auf Raumtemperatur erwärmen und läßt solange Rühren, bis keine Peroxide mehr nachweisbar sind. Man engt im Vakuum bis zur Trockne ein, kristallisiert aus Essigester um und erhält 0,785 g (78 % der Theorie) der Titelverbindung.
Schmelzpunkt: 181 °C.
0,748 g (1,94 mmol) der Verbindung aus b) und 1,54g (11,1 mmol) $K_2CO_3$ werden unter Argon mit 30 ml DMF versetzt. Nach 1 h werden 70 ml Wasser addiert und die Suspension 30 Minuten gerührt. Der Feststoff wird abgesaugt, zweimal mit Wasser und zweimal mit Ether gewaschen und im Hochvakuum getrocknet. Man erhält 0,62 g (88 % der Theorie) der Titelverbindung (Beispiel I).
Schmelzpunkt: 245 °C.

Beispiel II

6,7,8-Trifluor-4-oxo-1-(4-phenyl-but-1-en-3-yl)-1,4-dihydro-chinolin-3-carbonsäure-ethylester

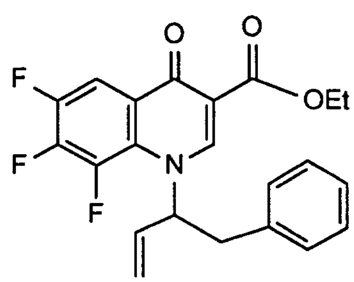

Zu einer Lösung von 4,57 g (14,3 mmol) 3-Ethoxy-2-(2,3,4,5-tetrafluorbenzoyl)-acrylsäureethylester in 20 ml Ethanol tropft man bei 0 °C eine Lösung von 2,10 g (14,3 mmol) 3-Amino-4-phenyl-1-buten. Man rührt 2 h bei Raumtemperatur, engt anschließend im Vakuum ein und trocknet im Hochvakuum. Der Rückstand wird in 20 ml wassertreiem Dimethoxyethan gelöst und bei 0 °C zu einer Suspension von 602 mg (80 Gew.-%, 20 mmol) Natriumhydrid in 50 ml wasserfreiem Dimethoxyethan getropft. Man rührt 1 h bei 0 °C, versetzt mit 4,5 ml Eisessig und engt im Vakuum ein. Der Rückstand wird zwischen Methylenchlorid und Wasser verteilt. Man trocknet die organische Phase mit Natriumsulfat, engt ein und chromatographiert an Kieselgel mit Toluol/Essigester.
Ausbeute: 3,63 g (63 %).

Beispiel III

1-(1-Benzyloxy-1-buten-3-yl)-6,7,8-trifluor-4-chinolon-3-carbonsäure-ethylester

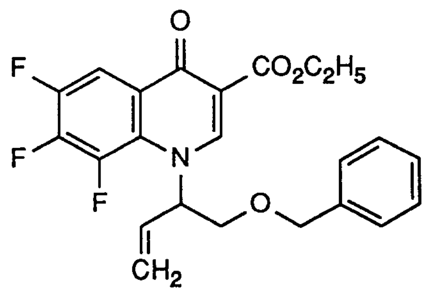

Zu einer Lösung von 26,25 g (82 mmol) 3-Ethoxy-2-(2,3,4,5-tetrafluorbenzoyl)-acrylsäureethylester in 105 mi Ethanol gibt man bei 0 °C eine Lösung von 14,5 g (82 mmol) 3-Amino-4-benzyloxy-1-buten. Man rührt 2 h bei Raumtemperatur, engt anschließend im Vakuum ein und trocknet im Hochvakuum. Der Rückstand wird in 200 ml wasserfreiem Dimethoxyethan gelöst und bei 0 °C zu einer Suspension von 2,93 g (97 mmol) Natriumhydrid (80%ig in Öl) in 235 ml wasserfreiem Dimethoxyethan getropft. Man rührt 1 h bei 0 °C, versetzt mit 22 ml Eisessig und engt im Vakuum ein. Der Rückstand wird zwischen Methylenchlorid und Wasser verteilt. Man trocknet die organische Phase mit Natriumsulfat, engt ein und chromatographiert an Kieselgel mit Toluol/Essigester.
Ausbeute: 15g (42 %)
Schmelzpunkt: 61-63 °C.

Beispiel IV

3-Benzyl-9,10-difluor-7-oxo-7H-pyrido[1,2,3-de][1,4]benzoxazin-6-carbonsäureethylester

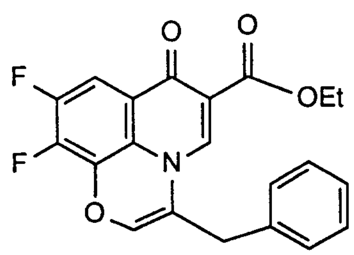

In eine Lösung von 3,6 g (9,0 mmol) der Verbindung aus Beispiel II in 80 ml Methanol und 20 ml Methylenchlorid leitet man bei -78 °C Ozon bis zur beginnenden Blaufärbung. Überschüssiges Ozon wird mit Stickstoff vertrieben. Man gibt 7,1 ml Dimethylsulfid bei -78 °C zu und läßt auf Raumtemperatur kommen. Man engt im Vakuum ein, nimmt den Rückstand in 50 ml wasserfreiem DMF auf und versetzt mit 1,25 g (9,0 mmol) Kaliumcarbonat. Nach 1 h bei Raumtemperatur fügt man 5 ml Eisessig sowie anschließend 500 ml Wasser, saugt den ausgefallenen Niederschlag ab und trocknet im Hochvakuum.
Ausbeute: 3,3 g (95 %); Schmelzpunkt: >260 °C.

Beispiel V

3-Benzyloxymethyl-9,10-difluor-7-oxo-7H-pyrido[1,2,3-de][1,4]benzoxazin-6-carbonsäureethylester

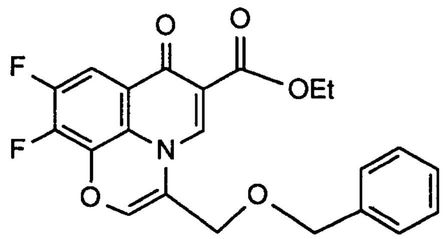

In Analogie zur Vorschrift des Beispiels IV wird die Titelverbindung durch Einsatz der Verbindung aus Beispiel III hergestellt.
Ausbeute: 45 %.

Beispiel VI

3-Benzyl-9,10-difluor-7-oxo-7H-pyrido[1,2,3-de][1,4]benzoxazin-6-carbonsäure

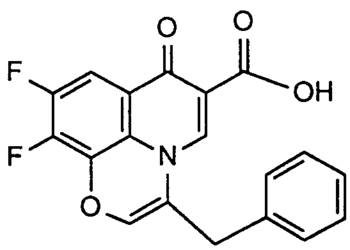

3,3 g (8,6 mmol) der Verbindung aus Beispiel IV, 47 ml Eisessig, 41 ml Wasser und 1 ml konzentrierte Schwefelsäure werden zusammen 4 h unter Rückfluß erhitzt. Nach dem Abkühlen versetzt man mit Wasser, saugt den ausgefallenen Niederschlag ab und trocknet ihn im Hochvakuum.
Ausbeute: 2,5 g (81 %); Schmelzpunkt: >260 °C.

Beispiel VII

3-Benzyloxymethyl-9,10-difluor-7-oxo-7H-pyrido[1,2,3-de][1,4]benzoxazin-6-carbonsäure

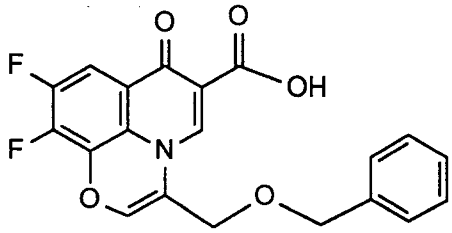

In Analogie zur Vorschrift des Beispiels VI wird durch Umsetzung der Verbindung aus Beispiel V die Titelverbindung hergestellt.
Ausbeute: 77 %; Schmelzpunkt: 168-170 °C.

Beispiel VIII

9,10-Difluor-2-(2-methyl-5-phenoxy-2-pentyl)-7-oxo-7H-pyrido[1,2,3-de][1,4]-benzoxazin-6-carbonsäureethylester

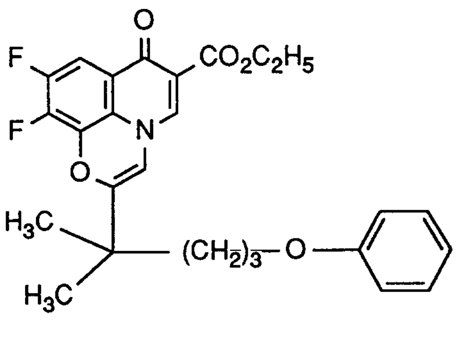

Unter Argon werden 2 g (7,38 mmol) 6,7,8-Trifluor-4-hydroxy-3-chinoloncarbonsäureethylester in 20 ml abs. DMF suspendiert. Es werden 2,55 g (18,44 mmol) $K_2CO_3$ und 3,76 g (14,75 mmol) 1-Chlor-3,3-dimethyl-6-phenoxy-2-hexanon in 20 ml abs. DMF zugegeben und 6 h auf 80 ° C erhitzt. Es wurden weitere 1,8 g (7,38 mmol) Chlorketon addiert und weitere 14 h auf 80 ° C erhitzt. Nach Abkühlen wurde der Ansatz auf 400 ml Wasser gegeben und mit 1N HCl angesäuert Man extrahiert mehrmals mit $CH_2Cl_2$, wäscht die organische Phase mit Wasser, trocknet über $Na_2SO_4$ und engt ein. Man löst in wenig $CH_2Cl_2$: MeOH = 100:3 und reinigt über eine Kieselgelsäule (gleiches Laufmittel).
Ausbeute: 1,15 g (34 % der Theorie); Schmelzpunkt: 96 ° C.

Beispiel IX

2-(1,1-Dimethyl-4-phenoxybutyl)-9,10-difluor-7-oxo-7H-pyrido[1,2,3-de][1,4]benzoxazin-6-carbonsäure

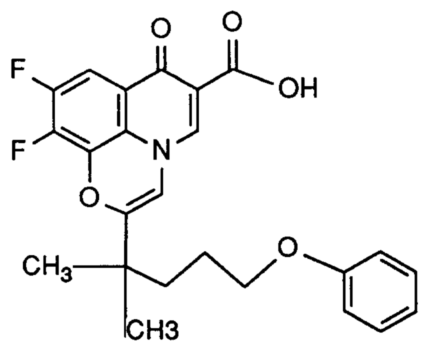

1,1 g (2,34 mmol) der Verbindung aus Beispiel VIII werden in einem Gemisch aus 1,5 mi $H_2SO_4$, 12 ml $H_2O$ und 18 ml Essigsäure suspendiert und erhitzt 4 h auf 120 ° C. Man läßt abkühlen, saugt den Niederschlag ab, wäscht mit Wasser nach und trocknet im Hochvakuum. Man erhält 0,89 g (86 % der Theorie) der Titelverbindung.
Schmelzpunkt: 212 ° C.

Beispiel X

9,10-Difluor-2-(2-amino-3-chloro-5-pyridyl)-7-oxo-7H-pyrido[1,2,3-de][1,4]benzoxazin-6-carbonsäure

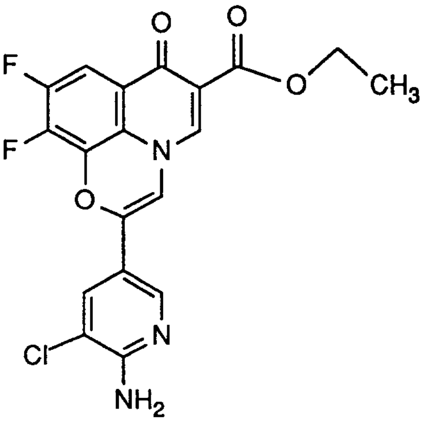

Unter Argon gibt man zu 2 g (7,38 mmol) 6,7,8-Trifluor-4-hydroxy-3-chinoloncarbonsäureethylester in 20 ml abs. DMF 1,55 g (18,44 mmol) $K_2CO_3$. Dazu addiert man eine Lösung von 3,7 g (14,75 mmol) 1-Brom-2-(2-amino-3-chloro-5-pyridyl)-2-ethanon in 20 ml abs. DMF und erhitzt 8 h auf 90 °C. Das Lösemittel wird im Vakuum entfernt, der Feststoff mit Wasser verrührt und abgesaugt. Der Rückstand wird mit Aceton aufgenommen und eingeengt bis zur Kristallbildung. Das Rohprodukt wird abgesaugt und an Kieselgel (Laufmittel $CH_2Cl_2$:MeOH = 100:5) gereinigt. Man erhält 2,58 g (83 % der Theorie) der Titelverbindung.
Schmelzpunkt: 200 °C (Zersetzung).

Beispiel XI

9,10-Difluor-2-(2-amino-3-chloro-5-pyridyl)-7-oxo-7H-pyrido[1,2,3-de][1,4]benzoxazin-6-carbonsäure

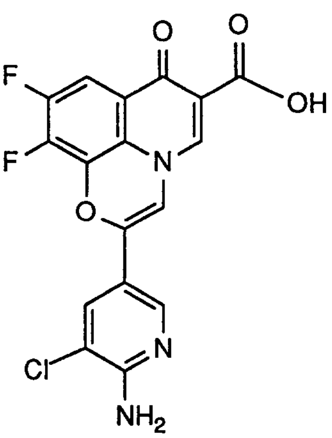

Zu 2,5 g (5,96 mmol) der Verbindung aus Beispiel X gibt man 12 ml Essigsäure, 8 ml Wasser und 3 mi $H_2SO_4$. Die Suspension wird 2 h auf 120 °C erhitzt. Nach Abkühlen wird mit Wasser verdünnt, der Feststoff abgesaugt und mit Wasser neutral gewaschen. Das Produkt wird im Hochvakuum getrocknet. Man erhält 702 mg (30 % der Theorie) der Titelverbindung.
Schmelzpunkt: >230 °C.

Beispiel XII

3-(1-Imidazolylmethyl)-9,10-difluor-7-oxo-7H-pyrido[1,2,3-de][1,4]benzoxazin-6-carbonsäureethylester

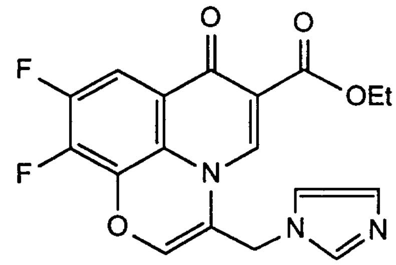

Zu 3,23 g (9,94 mmol) der Verbindung aus Beispiel XIII und 1,37 g (9,94 mmol) $K_2CO_3$ in 30 ml DMF gibt man 1,35 g (19,89 mmol) Imidazol und rührt 2,5 h bei Raumtemperatur. Man versetzt mit Wasser, saugt das Produkt ab, wäscht mit Wasser und Petrolether nach und trocknet im Hochvakuum. Man erhält 3,41 g (92 % der Theorie) der Titelverbindung.

Beispiel XIII

6,7,8-Trifluor-1-(2-oxo-1-methylen-ethyl)-4-chinolon-3-carborsäure-ethylester

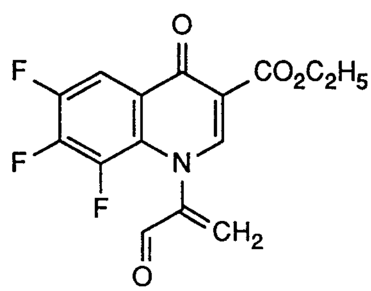

In eine Lösung von 30,85 g (71,5 mmol) der Verbindung aus Beispiel III in 640 ml Methanol und 160 ml Methylenchlorid leitet man bei -78 ° C Ozon bis zur Blaufärbung ein. Überschüssiges Ozon wird mit Stickstoff bei -78 ° C vertrieben. Man fügt 57 ml Dimethylsulfid zu, läßt auf Raumtemperatur kommen und rührt über Nacht bei dieser Temperatur. Nach dem Einengen im Vakuum chromatographiert man an Kieselgel mit Methylenchlorid/Methanol.
Ausbeute: 22,1 g (97%)
Schmelzpunkt: 185-187 ° C.

Beispiel XIV

2-Ethoxycarbonyl-3-(9,10-difluor-6-ethoxycarbonyl-7-oxo-7H-pyrido[1,2,3-de]-[1,4]benzoxazin3-yl)-propionsäure-ethylester

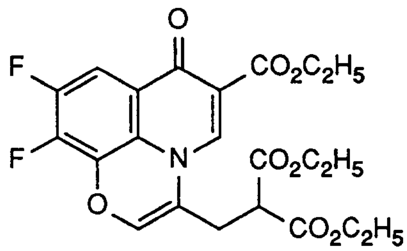

Zu einer Suspension von 329 mg (10,9 mmol) Natriumhydrid (80%ig in Öl) in 55 ml wasseifreiem DMF gibt man 1,75 g (10,9 mmol) Malonsäure-diethylester. Sobald eine klare Lösung entstanden ist, fügt man 3,55 g (10,9 mmol) der Verbindung aus Beispiel XIII zu und rührt 4 h bei Raumtemperatur. Man fügt 5,5 ml Eisessig und 500 mi Wasser zu, verrührt den entstandenen Niederschlag über Nacht, saugt ab und wäscht mit Wasser. Nach Chromatographie an Kieselgel mit Methylenchlorid/Methanol erhält man 3,44 g (68%) der Titelverbindung.

Beispiel XV

3-(6-Carboxy-9,10-difluor-7-oxo-7H-pyrido[1,2,3-de][1,4]benzoxazin-3-yl)-propionsäure

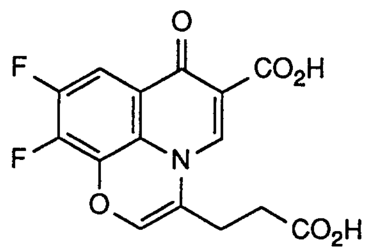

400 mg (0,86 mmol) der Verbindung aus Beispiel XIV werden in 12 ml Eisessig, 10 ml Wasser und 1 ml Schwefelsäure 4 h unter Rückfluß erhitzt. Nach dem Abkühlen versetzt man mit 100 ml Wasser, saugt den entstandenen Niederschlag ab, wäscht mit Wasser und trocknet im Hochvakuum.
Ausbeute: 271 mg (94%)
Schmelzpunkt: 252-254 ° C.

Beispiel XVI

1-(1-Cyclopropyl-2-propenyl)-6,7,8-trifluor-4-oxo-1,4-dihydro-chinolin-3-carbonsäure-ethylester

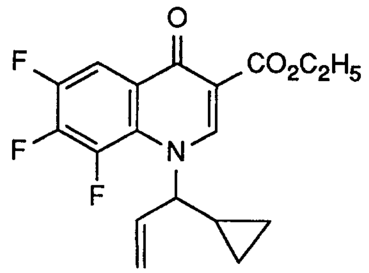

Die Titelverbindung wird in Analogie zur Vorschrift des Beispiels III aus 1-Amino-1-cyclopropyl-2-propen hergestellt.

Beispiel XVII

3-Cyclopropyl-9,10-difluor-7-oxo-7H-pyrido[1,2,3-de][1,4]benzoxazin-6-carbonsäure-ethylester

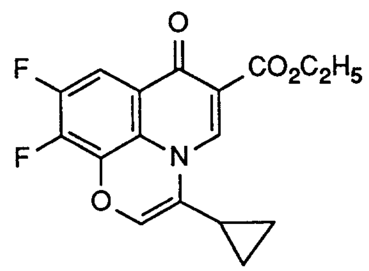

Die Verbindung wird in Analogie zur Vorschrift des Beispiels IV aus der Verbindung des Beispiels XVI hergestellt.

Beispiel XVIII

3-Cyclopropyl-9,10-difluor-7-oxo-pyrido[1,2,3-de][1,4]benzoxazin-6-carbonsäure

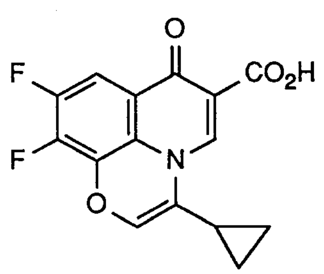

Die Titelverbindung wird in Analogie zur Vorschrift des Beispiels VI aus der Verbindung des Beispiels XVII hergestellt.

Beispiel XIX

Brommethylcyclopropylketon

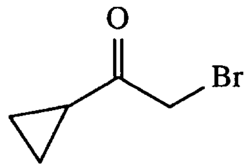

10,5 g (0,125 mol) Cyclopropylmethylketon werden in 100 ml Methanol gelöst und auf 0 °C gekühlt. Man addiert in einer Portion 19,95 g (0,125 mol) Brom und rührt bei +10 °C weiter, bis sich die Lösung fast vollständig entfärbt hat. Man erwärmt auf Raumtemperatur, rührt weitere 30 min und setzt 40 ml $H_2O$ zu. Man rührt nochmals 15 min und gibt den Ansatz auf 120 ml $H_2O$. Man extrahiert viermal mit je 100 ml Ether. Die organischen Phasen werden vereinigt und zweimal mit 10 %iger $K_2CO_3$-Lösung gewaschen. Man wäscht anschließend mit ges. NaCl-Lösung, trocknet über $MgSO_4$ und engt am Vakuum ein.

Das bräunliche Rohprodukt wird destilliert und man erhält 12,2 g (60 % der Theorie) der Titelverbindung.
$Kp_{12mm}$ = 80-90 °C.

Beispiel XX

2-Cyclopropyl-9,10-difluor-7-oxo-7H-pyrido[1,2,3-de][1,4]benzoxazin-6-carbonsäureethylester

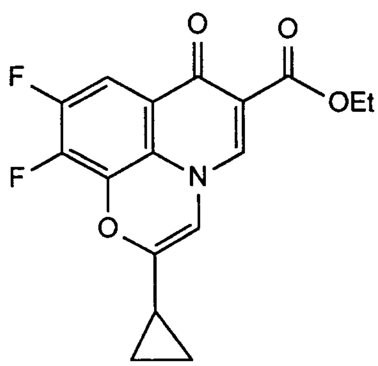

Unter Argon werden 5 g (18,4 mmol) 6,7,8-Trifluor-4-hydroxy-3-chinolin-carbonsäureethylester in 100 ml abs. DMF suspendiert. Man gibt 6,37 g (46,1 mmol) $K_2CO_3$ und 6,0 g (36,9 mmol) Brommethylcyclopropylketon zu und erhitzt 12 h auf 90 °C. Man destilliert das DMF im Vakuum ab. Der Rückstand wird mit $CH_2Cl_2$ aufgenommen und dreimal mit je 150 ml 1N HCl gewaschen. Die organische Phase wird mit $H_2O$ neutral gewaschen, über $MgSO_4$ getrocknet und eingeengt. Das Rohprodukt wird säulenchromatographisch an Kieselgel (Laufmittel $CH_2Cl_2$:MeOH: $NH_{3(aq)}$ = 100:2:1 gereinigt. Man nimmt mit Toluol auf und filtriert. Nach erneutem Einengen wird aus Essigester umkristallisiert und man erhält 2,1 g (34 % der Theorie) der Titelverbindung.
Schmelzpunkt: 212 °C.

Beispiel XXI

2-Cyclopropyl-9,10-difluor-7-oxo-7H-pyrido[1,2,3-de][1,4]benzoxazin-6-carbonsäure

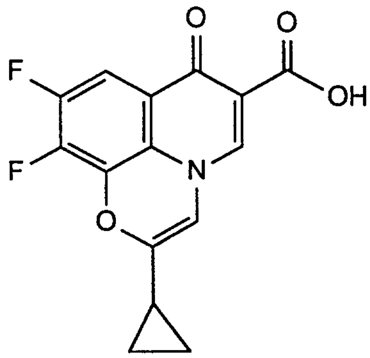

200 mg (0,6 mmol) der Verbindung aus Beispiel XX werden mit 72 mg (3 mmol) LiOH und einem Gemisch aus 3 ml THF und 1 ml $H_2O$ versetzt und 90 min bei 40 °C gerührt. Man stellt mit HCl auf pH 1, kühlt auf 0 °C und saugt ab. Der Rückstand wird mit $H_2O$ neutral gewaschen und im Vakuum getrocknet. Man erhält 130 mg (71 % der Theorie) der Titelverbindung.
Schmelzpunkt: >230 °C.

Beispiel XXII

3-(1-Imidazolylmethyl)-9,10-difluor-7-oxo-7H-pyrido[1,2,3-de][1,4]benzoxazin-6-carbonsäure-hydrochlorid

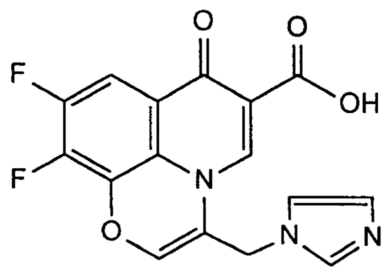

x HCl

Zu 3,41 g (9,13 mmol) der Verbindung aus Beispiel XII gibt man 340 ml 2N HCl und erhitzt 4 h unter Rückfluß. Man filtriert heiß, läßt die Lösung abkühlen, saugt die gebildeten Kristalle ab und trocknet im Hochvakuum.
Man erhält so 2,92 g (84 % der Theorie) der Titelverbindung.

Herstellungsbeispiele

Beispiel 1

9-Fluor-3-methyl-10-(4-(2-chlorphenyl)piperazin-1-yl)7-oxo-7H-pyrido[1,2,3-de]-[1,4]benzoxazin-6-carbonsäure

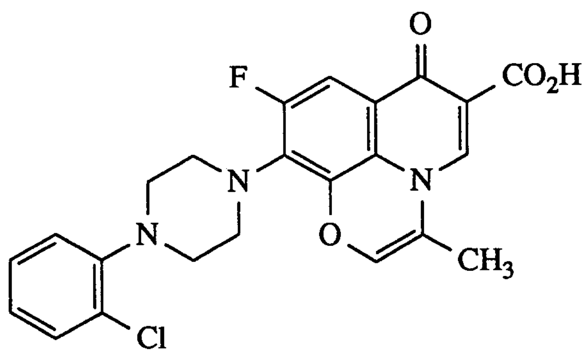

300 mg (1,075 mmol) 9,10-Difluor-3-methyl-7-oxo-7H-pyrido[1,2,3-de][1,4]benzoxazin-6-carbonsäure werden in 8 ml DMSO suspendiert und mit 520 mg (3,23 mmol) N-(2-Chlorphenyl)piperazin versetzt und 2 h bei 100 ° C erhitzt. Das Lösemittel wird im Kugelrohrvakuum entfernt (Badtemperatur 80 ° C), der Rückstand mit Ether verrührt, abgesaugt und der Feststoff anschließend mit Isopropanol verrührt, abgesaugt und getrocknet.
Ausbeute 322 mg (95% der Theorie); F: >265 ° C.

In Analogie zur Vorschrift des Beispiels 1 werden die in Tabellen 1 und 2 aufgeführten Verbindungen hergestellt:

Tabelle 1:

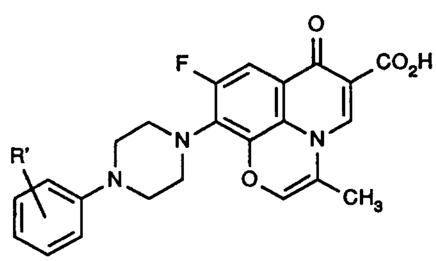

| Bsp.-Nr. | R' | F°C | Ausbeute (% d.Th.) |
|---|---|---|---|
| 2 | m-$OCH_3$ | 237 | 57 |
| 3 | o-$C_2H_5$ | 280 | 56 |
| 4 | o-$OC_2H_5$ | 268 | 74 |
| 5 | p-F | 260 | 85 |
| 6 | H | 224 | 72 |

Tabelle 2:

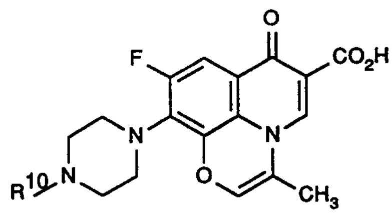

| Bsp.-Nr. | $R^{10}$ | F°C | Ausbeute (% d.Th.) |
|---|---|---|---|
| 7 | 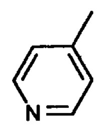 | Zers. | 45 |
| 8 | 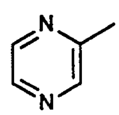 | 273 | 55 |

Beispiel 9

10-(4-Cyclopropyl-piperazin-1-yl)-9-fluor-3-methyl-7-oxo-7H-pyrido-[1,2,3-de][1,4]benzoxazin-6-carbonsäure

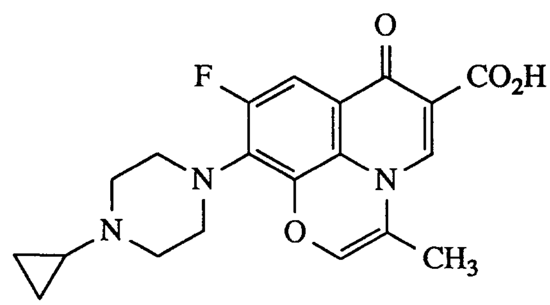

300 mg (1,075 mmol) 9,10-Difluor-3-methyl-7-oxo-7H-pyrido[1,2,3-de][1,4]benzoxazin-6-carbonsäure werden in 8 ml DMSO suspendiert, mit 139,8 mg (1,075 mmol) 1-Cyclopropylpiperazin und 240,8 mg (2,15 mmol) DABCO versetzt und 1 h bei 100 ° C erhitzt. Nach vollständiger Umsetzung wird das Lösemittel im Kugelrohrvakuum abdestilliert, der Rückstand mit Isopropanol aufgeschlämmt und der Feststoff abgesaugt und getrocknet.

Ausbeute: 398 mg (78% der Theorie) als Feststoff; F: >265 ° C.

In Analogie zur Vorschrift des Beispiels 9 werden die in den Tabellen 3,4 und 5 aufgeführten Verbindungen erhalten:

<u>Tabelle 3:</u>

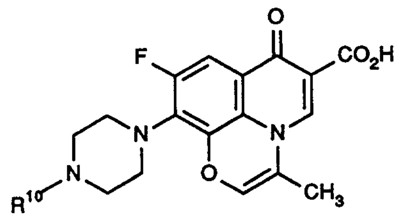

| Bsp.-Nr. | $R^{10}$ | F°C | Ausbeute (% d.Th.) |
|---|---|---|---|
| 10 | $-CH_2CF_3$ | 255 | 87 |

<u>Tabelle 4:</u>

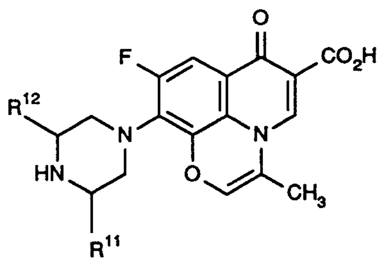

| Bsp.-Nr. | $R^{11}$ | $R^{12}$ | F°C | Ausbeute (% d.Th.) |
|---|---|---|---|---|
| 11 | H | $-CH_2-OC_6H_5$ | Zers. | 50 |
| 12 | H | $-(CH_2)_2OH$ | 183 | 95 |
| 13 | H | 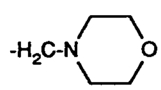 | Zers. | 22 |

Tabelle 5:

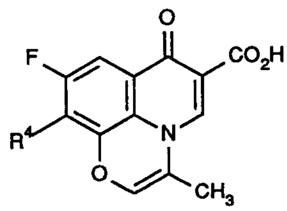

| Bsp.-Nr. | $R^4$ | F°C | Ausbeute (% d.Th.) |
|---|---|---|---|
| 14 | 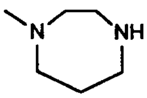 | 210 | 91 |
| 15 | 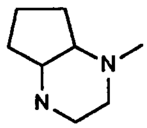 | 195 | 93 |
| 16 | 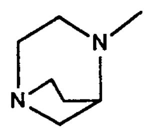 | Zers. | 47 |
| 17 | 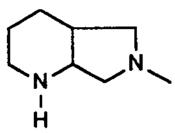 | >265 | 64 |
| 18 | 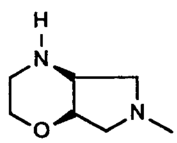 | >265 | 95 |
| 19 | 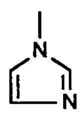 | >265 | 78 |

Beispiel 20

3-Benzyl-9-fluor-7-oxo-10-(1-piperazinyl)-7H-pyrido[1,2,3-de][1,4]benzoxazin-6-carbonsäure

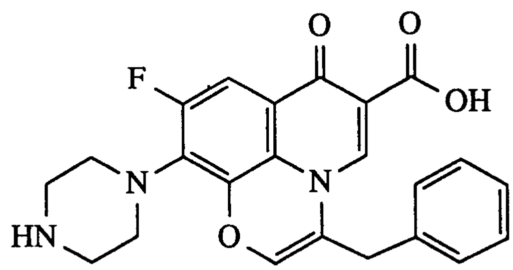

355 mg (1,0 mmol) der Verbindung aus Beispiel VI, 5 ml DMSO, 1,7 ml Diisopropylethylamin (10 mmol) und 172 mg (2,0 mmol) Piperazin werden 4 h unter Rückfluß erhitzt. Nach dem Abkühlen versetzt man mit Ether und saugt den ausgefallenen Niederschlag ab.
Ausbeute: 290 mg (69%); Schmelzpunkt: 169 °C (Zers.).

In Analogie zur Vorschrift des Beispiels 20 werden die in Tabelle 6 aufgeführten Verbindungen hergestellt.

Tabelle 6:

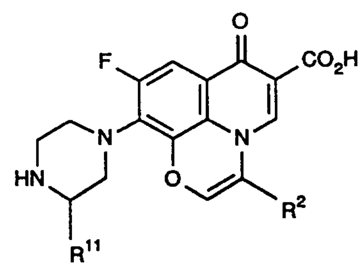

| Bsp.-Nr. | $R^2$ | $R^{11}$ | F°C | Ausbeute (% der Theorie) |
|---|---|---|---|---|
| 21 | $-CH_2-C_6H_5$ | $-CH_3$ | 248-250 (Zers.) | 36 |
| 22 | $-CH_2-O-CH_2-C_6H_5$ | H | 228-230 (Zers.) | 63 |
| 23 | $-CH_2-O-CH_2-C_6H_5$ | $-CH_3$ | 186 (Zers.) | 44 |

Beispiel 24

2-(1,1-Dimethyl-4-phenoxy-butyl)-9-fluor-10-piperazinyl-7-oxo-7H-pyrido-[1,2,3-de][1,4]benzoxazin-6-carbonsäure

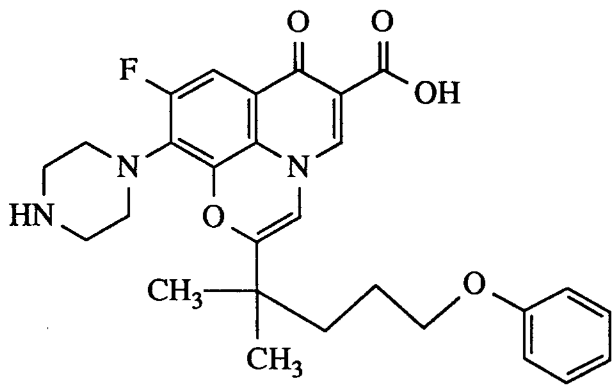

Unter Argon werden 0,2 g (0,453 mmol) der Verbindung aus Beispiel IX in 2 ml absolutem DMF suspendiert. Man gibt 0,11 g (0,906 mmol) Diisopropylethylamin und 0,12 g (1,36 mmol) Piperazin zu und erhitzt 4h auf 120 °C.

Man destilliert das Lösungsmittel ab, versetzt den Rückstand mit Ether und saugt das Produkt ab. Man wäscht mit Wasser und trocknet im Hochvakuum.

Beispiel 25

9-Fluor-2-(1,1-dimethyl-4-phenoxy-butyl)-10-(3-methyl-1-piperazinyl)-7-oxo-7H-pyrido[1,2,3-de][1,4]-benzoxazin-6-carbonsäure

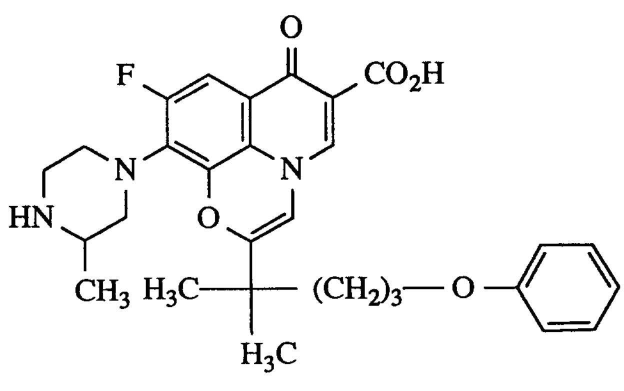

In Analogie zu Beispiel 24 wird die Titelverbindung mit 2-Methylpiperazin hergestellt.

Die in Tabelle 7 aufgeführten Beispiele werden in Analogie zu den dort angegebenen Verbindungen hergestellt:

Tabelle 7:

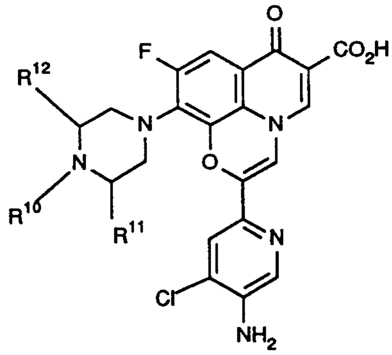

| Bsp.-Nr. | $R^{10}$ | $R^{11}$ | $R^{12}$ | F°C | Ausbeute (% d. Th.) | in Analogie zu Beispiel |
|---|---|---|---|---|---|---|
| 26 | $-CH_3$ | H | H | >230 | 60 | 24 |
| 27 | H | $-CH_3$ | H | >230 | 67 | 24 |
| 28 | H | H | H | >230 | 59 | 24 |
| 29 | H | $-CH_3$ | $-CH_3$ | >230 | 40 | 24 |

Beispiel 30

9-Fluor-10-(4-methyl-1-piperazinyl)-7-oxo-7H-pyrido[1,2,3-de][1,4]benzoxazin-2,7-dicarbonsäureethylester

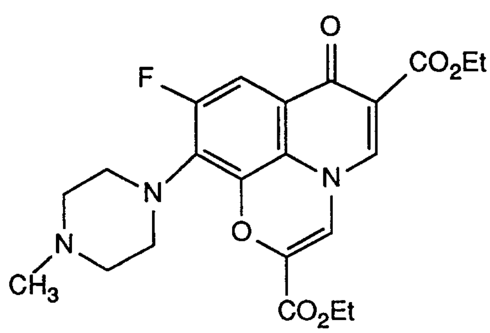

0,5 g (1,37 mmol) der Verbindung aus Beispiel I, 0,41 g (4,1 mmol) N-Methylpiperazin und 0,35 g (2,74 mmol) Hünigbase werden mit 15 ml DMSO versetzt und unter Argon 4 h bei 120 °C gerührt. Man engt im Vakuum ein und reinigt das Rohprodukt durch Chromatographie an Kieselgel ($CH_2Cl_2$:MeOH:$NH_{3(aq)}$ = 100:2,5:1). Man erhält 0,116 g (19 % der Theorie) der gewünschten Verbindung.
Schmelzpunkt: 168-170 °C.

Beispiel 31

9-Fluor-10-(4-methyl-1-piperazinyl)-7-oxo-7H-pyrido[1,2,3-de][1,4]benzoxazin-2,7-dicarbonsäure

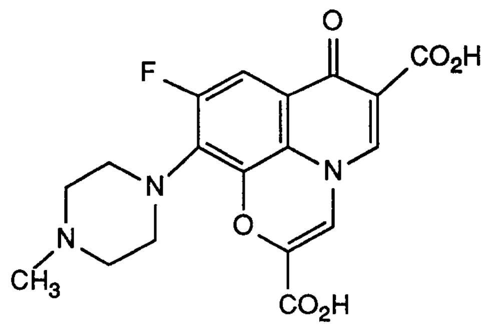

104 mg (0,233 mmol) der Verbindung aus Beispiel 30 werden in 5 ml eines $HOAc/H_2O/H_2SO_4$-Gemisches (12:8:1) 12 Stunden auf 120 °C erhitzt. Man saugt den Feststoff ab und trocknet im Hochvakuum. Man erhält so 52 mg (57 % der Theorie) der Titelverbindung.
Schmelzpunkt: >230 ° C.
$^1$HNMR(DMSO-d6): 8,95 (s 1H); 7,97 (s 1H); 7,49 (d J = 11,5; 1H); 3,7-3,0 (m, 8H); 2,85 (s 3H).

In Analogie zur Vorschrift des Beispiels 20 werden die in Tabelle 8 aufgeführten Verbindungen hergestellt:

Tabelle 8:

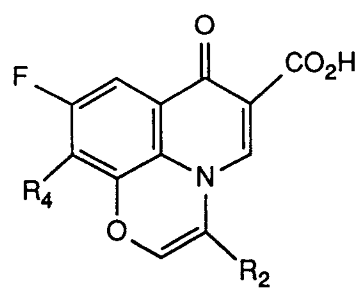

| Bsp.Nr. | $R^4$ | $R^2$ |
|---|---|---|
| 32 | 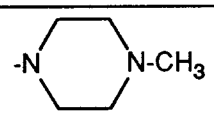 | $-CH_2-C_6H_5$ |
| 33 | 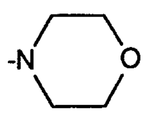 | $-CH_2-C_6H_5$ |
| 34 | 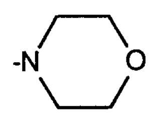 | $-(CH_2)_2-CO_2H$ |

Die in Tabelle 9 aufgeführten Verbindungen werden in Analogie zur Vorschrift des Beispiels 20 aus der Verbindung des Beispiels XVIII hergestellt.

Tabelle 9:

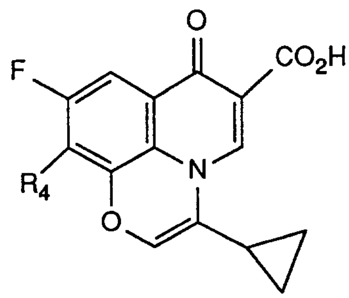

| Bsp.Nr. | $R^4$ |
|---|---|
| 35 | 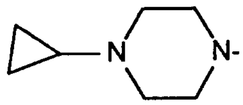 |
| 36 | 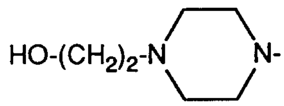 |
| 37 | 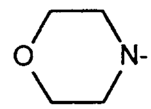 |
| 38 | 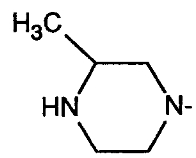 |
| 39 | 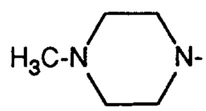 |
| 40 | 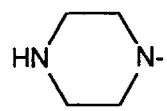 |

Beispiel 41

2-Cyclopropyl-9-fluor-10-(3-methyl-1-piperazinyl)-7-oxo-7H-pyrido[1,2,3-de][1,4]-benzoxazin-6-carbonsäureethylester

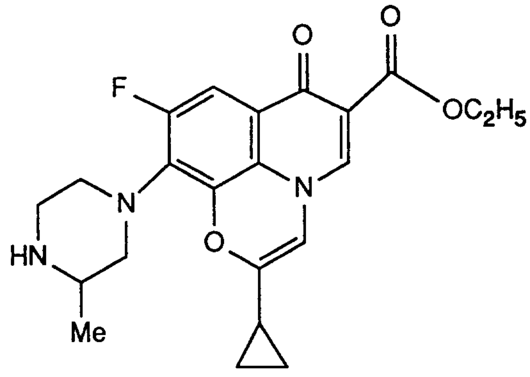

Unter Argon werden 0,5 g (1,5 mmol) 2-Cyclopropyl-9,10-difluor-7-oxo-7H-pyrido-[1,2,3-de][1,4]-benzoxazin-6-carbonsäureethylester in 10 ml abs. DMSO suspendiert. Man addiert 0,364 g (3 mmol) Diisopropylethylamin und 0,451 g (4,5 mmol) 2-Methylpiperazin und erhitzt 6h auf 120 ° C.
Man kühlt ab und zieht am Rotationsverdampfer DMSO ab. Der Rückstand wird in $CH_2Cl_2$ aufgenommen, mit gesättigter NaCl-Lösung geschüttelt, über $MgSO_4$ getrocknet und eingeengt. Das Rohprodukt wird an Kieselgel (Laufmittel $CH_2Cl_2$:MeOH:$NH_{3(aq)}$ = 100:2,5:1) gereinigt und liefert 300 mg (48 % der Theorie) der Titelverbindung.
Schmelzpunkt: amorph
$^1$HNMR ($CDCl_3$): 8,06 (s; 1H); 7,57 (d J = 12,3; 1H); 6,05 (s; 1H); 4,36 (q J = 7; 2H); 3,2-2,7(m); 1,52 (m; 1H); 1,39 (tr J = 7; 3H); 1,07 (d J = 6; 3H); 0,90 (m; 4H).

Beispiel 42

2-Cyclopropyl-9-fluor-10-(3-methyl-1-piperazinyl)-7-oxo-7H-pyrido[1,2,3-de][1,4]-benzoxazin-6-carbonsäurehydrochlorid

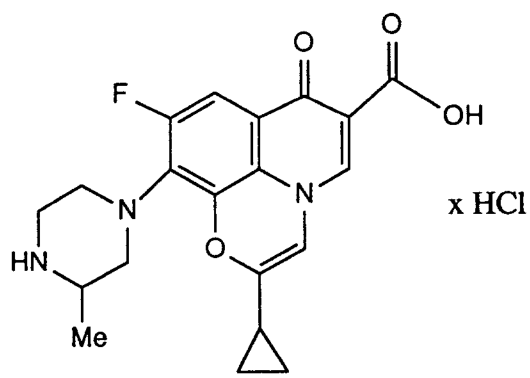

x HCl

180 mg (0,43 mmol) der Verbindung aus Beispiel 41 werden in einem Gemisch aus 6 ml THF und 2 ml $H_2O$ gelöst, mit 52 mg (2,17 mmol) LiOH versetzt und 6 h bei 40 ° C gerührt. Unter Eisbadkühlung wird auf pH 1 gestellt und der Niederschlag nach 30 min abgesaugt. Man wäscht mit kaltem Wasser neutral und trocknet im Hochvakuum. Man erhält 92 mg (55 % der Theorie) der gelben Titelverbindung.
Schmelzpunkt: >230 ° C.

Beispiel 43

2-Cyclopropyl-9-fluor-10-(4-methyl-1-piperazinyl)-7-oxo-7H-pyrido[1,2,3-de][1,4)-benzoxazin-6-carbonsäureethylester

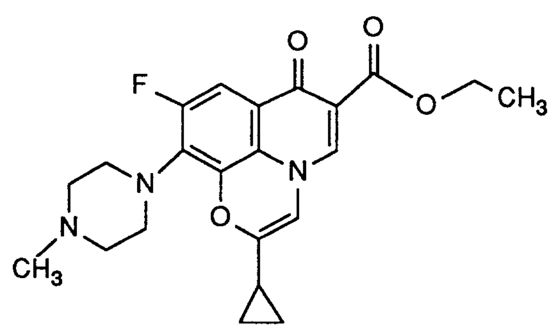

Analog zur Herstellung von Beispiel 41 erhält man in 52 % der Theorie die Titelverbindung.
Schmelzpunkt: 130 ° C.

Beispiel 44

2-Cyclopropyl-9-fluor-10-(4-methyl-1-piperazinyl)-7-oxo-7H-pyrido[1,2,3-de][1,4]-benzoxazin-6-carbonsäurehydrochlorid

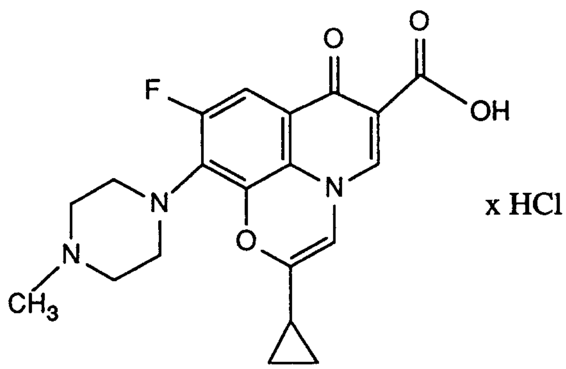

x HCl

238 mg (0,58 mmol) der Verbindung aus Beispiel 43 werden in 5 ml eines $CH_3CO_2H/H_2O/H_2SO_4$-Gemisches (12:8:1) gelöst und 4 Stunden auf 100 ° C erhitzt. Man entfernt das Lösungsmittel weitgehend, versetzt mit 1 mi konz. HCl und addiert 50 ml Ethanol. Man läßt über Nach rühren und saugt in der Kälte ab. Das 95 %ige Rohprodukt wird an Kieselgel (Laufmittel $CH_2Cl_2$:$CH_3OH$:$CH_3CO_2H$ = 100:25:4) gereinigt und erneut ins Hydrochlorid (siehe oben) überführt. Man trocknet im Hochvakuum und erhält 76 mg (32 % der Theorie) der Titelverbindung.

Schmelzpunkt: >230 ° C.

In Analogie zur Vorschrift des Beispiels 20 werden die in Tabelle 10 aufgeführten Beispiele hergestellt:

Tabelle 10:

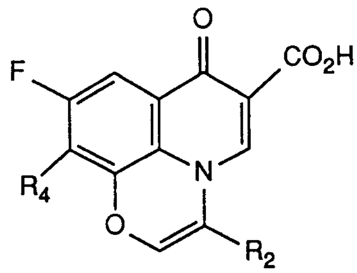

| Bsp.Nr. | $R^4$ | $R^2$ |
|---|---|---|
| 45 | 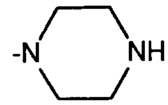 | 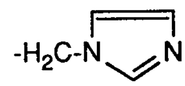 |
| 46 | 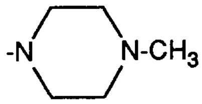 | 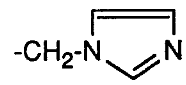 |
| 47 | 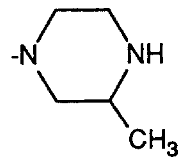 | 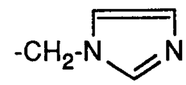 |
| 48 | 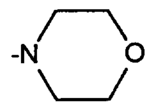 | 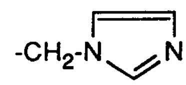 |
| 49 | 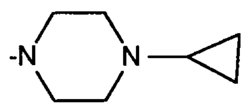 | 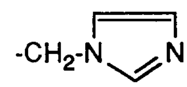 |
| 50 | 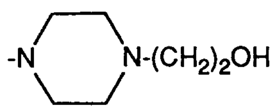 | 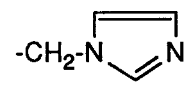 |

## Patentansprüche

**1.** 9-Fluor-7-oxo-7H-pyrido[1,2,3-de][1,4]benzoxazin-6-carbonsäure-Derivate der allgemeinen Formel

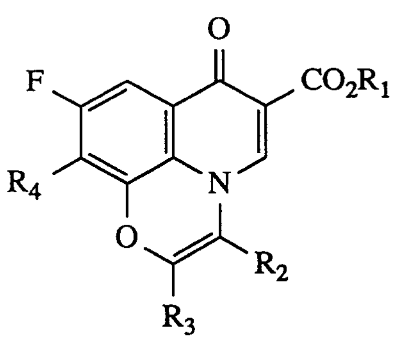

,

in welcher

$R^1$ für Wasserstoff oder geradkettiges oder verzweigtes Alkyl mit bis zu 8 Kohlenstoffato-

men steht,

$R^2$ für Wasserstoff, Formyl, Methyl, Benzyl, p-Cl-Benzyl, Carboxy oder für die $-CONH_2$-Gruppe steht, oder
für geradkettiges oder verzweigtes Alkenyl oder Alkoxycarbonyl mit jeweils bis zu 6 Kohlenstoffatomen steht, oder
für geradkettiges oder verzweigtes, durch Carboxy, Benzyloxy, Imidazolyl oder durch geradkettiges oder verzweigtes Alkoxycarbonyl mit bis zu 4 Kohlenstoffatomen substituiertes Alkyl mit bis zu 6 Kohlenstoffatomen steht, oder
für Cycloalkyl mit 3 bis 8 Kohlenstoffatomen steht,

$R^3$ für Wasserstoff,
für Carboxy oder
für Cycloalkyl mit 3 bis 8 Kohlenstoffatomen steht,
für geradkettiges oder verzweigtes Alkoxycarbonyl mit bis zu 8 Kohlenstoffatomen, oder
für geradkettiges oder verzweigtes, durch Carboxy, Phenoxy oder durch geradkettiges oder verzweigtes Alkoxycarbonyl mit bis zu 6 Kohlenstoffatomen substituiertes Alkyl mit bis zu 10 Kohlenstoffatomen steht, oder
für Biphenyl oder für einen Rest der Formel

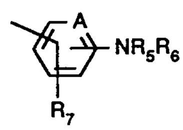

oder

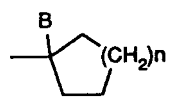

steht,
worin

A ein Stickstoffatom oder die -CH-Gruppe bedeutet,

$R^5$ und $R^6$ gleich oder verschieden sind und Wasserstoff oder geradkettiges oder verzweigtes Alkyl oder Acyl mit jeweils bis zu 6 Kohlenstoffatomen bedeuten,

$R^7$ Wasserstoff, Halogen, Hydroxy oder geradkettiges oder verzweigtes Alkoxy oder Alkyl mit jeweils bis zu 6 Kohlenstoffatomen bedeutet,

n eine Zahl 1,2 oder 3 bedeutet,
und

B geradkettiges oder verzweigtes Alkyl mit bis zu 6 Kohlenstoffatomen oder Wasserstoff bedeutet,

$R^4$ für Halogen oder über N-gebundenes Imidazolyl oder 1,4-Diazacycloheptanyl steht, oder
für einen Rest der Formel

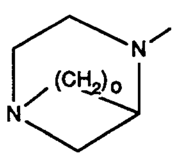

,

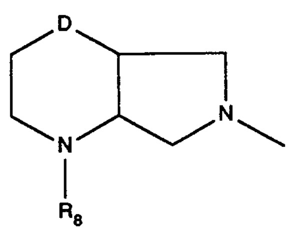

,

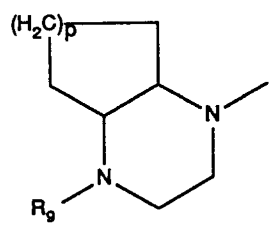

,

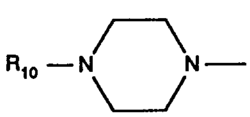

oder

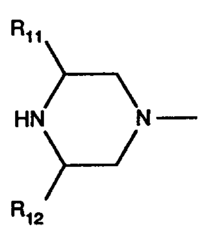

steht,

worin

o eine Zahl 2 oder 3 bedeutet,

p eine Zahl 1 oder 2 bedeutet,

D die $-CH_2$-Gruppe oder ein Sauerstoffatom bedeutet,

$R^8$ und $R^9$ gleich oder verschieden sind und Wasserstoff, Methyl oder Ethyl bedeuten,

$R^{10}$ Cycloalkyl mit 3 bis 8 Kohlenstoffatomen oder geradkettiges oder verzweigtes Perfluoralkyl mit bis zu 6 Kohlenstoffatomen bedeutet, oder
Phenyl, Pyridyl oder Pyrimidyl bedeutet, die gegebenenfalls durch Halogen oder geradkettiges oder verzweigtes Alkyl oder Alkoxy mit jeweils bis zu 6 Kohlenstoffatomen substituiert sind,

$R^{11}$ Wasserstoff oder Methyl bedeutet,

$R^{12}$ geradkettiges oder verzweigtes, durch Hydroxy, Benzyloxy, Phenoxy oder Morpholino substituiertes Alkyl mit bis zu 6 Kohlenstoffatomen bedeutet,

und im Fall, daß $R^3$ nicht Wasserstoff bedeutet, wenn $R^2$ für Wasserstoff oder Methyl steht,

$R^4$ außerdem für Morpholino oder für einen Rest der Formel

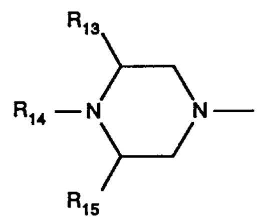

steht,
worin

$R^{13}$, $R^{14}$ und $R^{15}$ gleich oder verschieden sind und Wasserstoff, Hydroxyethyl, Methyl oder Ethyl bedeuten,

und deren Hydrate und Salze.

**2.** 9-Fluor-7-oxo-7H-pyrido[1,2,3-de][1,4]benzoxazin-6-carbonsäure-Derivate gemäß Anspruch 1, in welcher

$R^1$ für Wasserstoff, oder
für geradkettiges oder verzweigtes Alkyl mit bis zu 6 Kohlenstoffatomen steht,

$R^2$ für Wasserstoff, Formyl, Methyl, Benzyl, p-Cl-Benzyl, Carboxy oder
für die $-CONH_2$-Gruppe steht, oder
für geradkettiges oder verzweigtes Alkenyl oder Alkoxycarbonyl mit jeweils bis zu 4 Kohlenstoffatomen steht, oder
für geradkettiges oder verzweigtes, durch Carboxy, Benzyloxy, Imidazolyl, Methoxycarbonyl, Ethoxycarbonyl oder Propoxycarbonyl substituiertes Alkyl mit bis zu 4 Kohlenstoffatomen steht, oder
für Cyclopropyl, Cyclobutyl, Cyclopentyl oder Cyclohexyl steht,

$R^3$ für Wasserstoff,
für Carboxy, oder
für Cyclopropyl, Cyclobutyl, Cyclopentyl oder Cyclohexyl steht,
für geradkettiges oder verzweigtes Alkoxycarbonyl mit bis zu 6 Kohlenstoffatomen steht, oder
für geradkettiges oder verzweigtes, durch Carboxy, Phenoxy oder durch geradkettiges oder verzweigtes Alkoxycarbonyl mit bis zu 4 Kohlenstoffatomen substituiertes Alkyl mit bis zu 8 Kohlenstoffatomen steht, oder
für Biphenyl oder für einen Rest der Formel

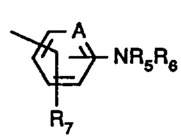

oder

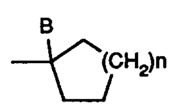

steht,
worin

A ein Stickstoffatom oder die -CH-Gruppe bedeutet,

$R^5$ und $R^6$ gleich oder verschieden sind und Wasserstoff oder geradkettiges oder verzweigtes Alkyl oder Acyl mit jeweils bis zu 4 Kohlenstoffatomen bedeuten,

$R^7$ Wasserstoff, Fluor, Chlor, Hydroxy oder geradkettiges oder verzweigtes Alkoxy oder Alkyl mit jeweils bis zu 4 Kohlenstoffatomen bedeutet,

n eine Zahl 1 oder 2 bedeutet,
und

B geradkettiges oder verzweigtes Alkyl mit bis zu 4 Kohlenstoffatomen oder Wasserstoff bedeutet,

$R^4$ für Fluor oder über N-gebundenes Imidazolyl oder 1,4-Diazacycloheptanyl steht, oder für einen Rest der Formel

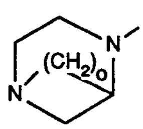 , 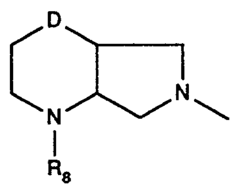 , 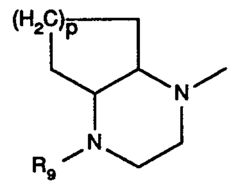 ,

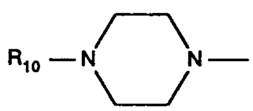 oder 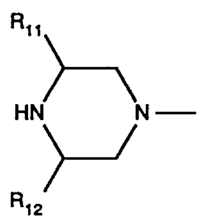

steht,

worin

o eine Zahl 2 oder 3 bedeutet,

p eine Zahl 1 oder 2 bedeutet,

D die $-CH_2$-Gruppe oder ein Sauerstoffatom bedeutet,

$R^8$ und $R^9$ gleich oder verschieden sind und Wasserstoff, Methyl oder Ethyl bedeuten,

$R^{10}$ Cyclopropyl, Cyclopentyl oder Cyclohexyl bedeutet, oder geradkettiges oder verzweigtes Perfluoralkyl mit bis zu 4 Kohlenstoffatomen bedeutet, oder
Phenyl, Pyridyl oder Pyrimidyl bedeutet, die gegebenenfalls durch Fluor, Chlor oder geradkettiges oder verzweigtes Alkyl oder Alkoxy mit jeweils bis zu 4 Kohlenstoffatomen substituiert sind,

$R^{11}$ Wasserstoff oder Methyl bedeutet,

$R^{12}$ geradkettiges oder verzweigtes, durch Hydroxy, Benzyloxy, Phenoxy oder Morpholino substituiertes Alkyl mit bis zu 4 Kohlenstoffatomen bedeutet,

und im Fall, daß $R^3$ nicht Wasserstoff bedeutet, wenn $R^2$ für Wasserstoff oder Methyl steht,

$R^4$ außerdem für Morpholino oder für einen Rest der Formel

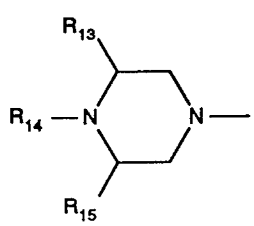

steht,

worin

$R^{13}$, $R^{14}$ und $R^{15}$ gleich oder verschieden sind und Wasserstoff, Hydroxyethyl, Methyl oder Ethyl bedeuten.

**3.** 9-Fluor-7-oxo-7H-pyrido[1,2,3-de][1,4]benzoxazin-6-carbonsäure-Derivate gemäß Anspruch 1, in welcher

$R^1$ für Wasserstoff oder für geradkettiges oder verzweigtes Alkyl mit bis zu 4 Kohlenstoffatomen steht,

$R^2$ für Wasserstoff, Formyl, Methyl, Benzyl, p-Cl-Benzyl, Carboxy oder für die $-CONH_2$-Gruppe steht,
für Vinyl, Allyl, Methoxycarbonyl oder Ethoxycarbonyl steht,
für geradkettiges oder verzweigtes, durch Carboxy, Benzyloxy, Imidazolyl, Methoxycarbonyl oder Ethoxycarbonyl substituiertes Alkyl mit bis zu 3 Kohlenstoffatomen steht, oder
für Cyclopropyl oder Cyclopentyl steht,

$R^3$ für Wasserstoff, oder
für Carboxy, oder
für Cyclopropyl oder Cyclopentyl steht oder
für geradkettiges oder verzweigtes Alkoxycarbonyl mit bis zu 4 Kohlenstoffatomen steht, oder
für geradkettiges oder verzweigtes, durch Carboxy, Phenoxy oder durch geradkettiges oder verzweigtes Alkoxycarbonyl mit bis zu 3 Kohlenstoffatomen substituiertes Alkyl mit bis zu 6 Kohlenstoffatomen steht, oder
für einen Rest der Formel

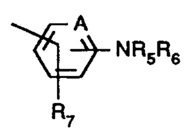

steht,
worin

A ein Stickstoffatom oder die -CH-Gruppe bedeutet,

$R^5$ und $R^6$ gleich oder verschieden sind und Wasserstoff, Methyl, Ethyl oder Acetyl bedeuten,

$R^7$ Wasserstoff, Fluor, Chlor, Methyl, Ethyl, Methoxy oder Ethoxy bedeutet,

$R^4$ für über N-gebundenes Imidazolyl oder 1,4-Diazacycloheptanyl steht, oder
für einen Rest der Formel

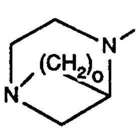 , 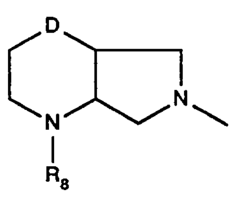 , 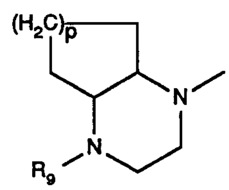 ,

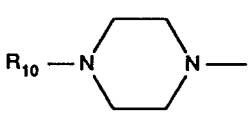 oder 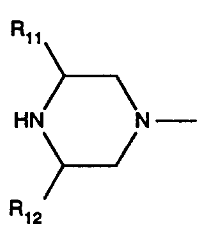

steht,

worin

| | |
|---|---|
| o | die Zahl 2 bedeutet, |
| p | eine Zahl 1 oder 2 bedeutet, |
| D | die $-CH_2$-Gruppe oder ein Sauerstoffatom bedeutet, |
| $R^8$ und $R^9$ | gleich oder verschieden sind und Wasserstoff, Methyl oder Ethyl bedeuten, |
| $R^{10}$ | Cyclopropyl oder geradkettiges oder verzweigtes Perfluoralkyl mit bis zu 3 Kohlenstoffatomen oder Phenyl, Pyridyl oder Pyrimidyl bedeutet, die gegebenenfalls durch Fluor, Chlor oder geradkettiges oder verzweigtes Alkyl oder Alkoxy mit jeweils bis zu 3 Kohlenstoffatomen substituiert sind, |
| $R^{11}$ | Wasserstoff oder Methyl bedeutet, |
| $R^{12}$ | geradkettiges oder verzweigtes, durch Hydroxy, Benzyloxy, Phenoxy oder Morpholin substituiertes, Alkyl mit bis zu 3 Kohlenstoffatomen bedeutet, |

und im Fall, daß $R^3$ nicht Wasserstoff bedeutet, wenn $R^2$ für Wasserstoff oder Methyl steht,

$R^4$ außerdem für Morpholino oder für einen Rest der Formel

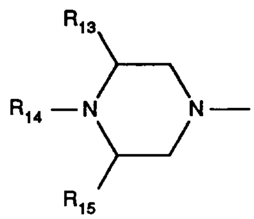

steht,

worin

$R^{13}$, $R^{14}$ und $R^{15}$ gleich oder verschieden sind und Wasserstoff, Hydroxyethyl, Methyl oder Ethyl bedeuten.

**4.** Verfahren zur Herstellung der Verbindungen gemäß Anspruch 1, dadurch gekennzeichnet, daß man

[A] Verbindungen der allgemeinen Formel (II)

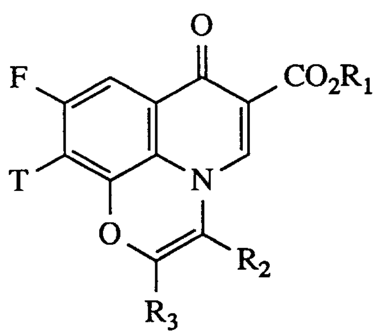

(II),

in welcher

$R^1$, $R^2$ und $R^3$ die in Anspruch 1 angegebene Bedeutung haben,

und

T für Halogen steht,

mit Verbindungen der allgemeinen Formel (III)

$R^4$-H (III),

in welcher

$R^4$ die oben angegebene Bedeutung hat,

in inerten Lösemitteln, in Anwesenheit einer Base unter Schutzgasatmosphäre umsetzt,

oder

Aldehyde der allgemeinen Formel (IV)

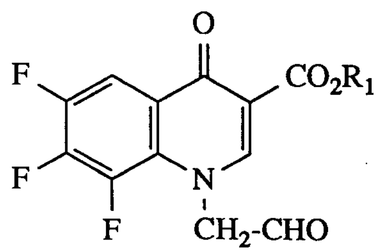

(IV),

in welcher
$R^1$ die oben angegebene Bedeutung hat,
zunächst mit Verbindungen der allgemeinen Formeln (III) oder (IIIa)

$R^4$-H (III),

W-H (IIIa),

in welcher
$R^4$ die oben angegebene Bedeutung hat,
W die oben angegebene Bedeutung von $R^4$ hat, wobei eine der cyclischen Aminfunktionen durch eine Schutzgruppe geschützt ist,
in inerten Lösemitteln, in Anwesenheit einer Base unter gleichzeitiger Cyclisierung umsetzt, die Schutzgruppe nach üblicher Methode abspaltet,
oder im Fall, daß $R^2$, wie oben definiert, für substituiertes Alkyl steht,
[B] Verbindungen der allgemeinen Formel (V)

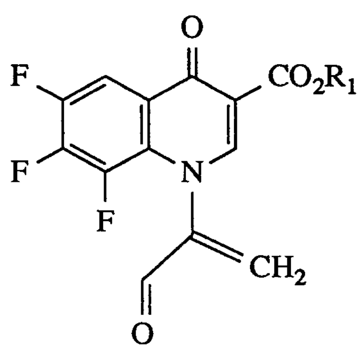

(V),

in welcher
$R^1$ die oben angegebene Bedeutung hat,
mit Verbindungen der allgemeinen Formel (VI)

$R^{17}$-H (VI),

in welcher
$R^{17}$ für das oben unter $R^2$ aufgeführte substituierte Alkyl steht, das um ein Kohlenstoffatom verkürzt ist,
in einem der oben aufgeführten Lösemitteln, gegebenenfalls in Anwesenheit einer Base unter gleichzeitiger Cyclisierung umsetzt, und in einem letzten Schritt den Substituenten $R^4$, wie oben beschrieben, einführt,
und im Fall, daß $R^3 \neq H$, derivatisiert,
und anschließend je nach gewünschter Bedeutung für $R^1$ entweder eine Veresterung oder Verseifung nach üblicher Methode durchführt.

**5.** 9-Fluor-7-oxo-7H-pyrido[1,2,3-de][1,4]benzoxazin-6-carbonsäure-Derivate gemäß Ansprüchen 1 bis 3, zur Bekämpfung von Krankheiten.

**6.** 9-Fluor-7-oxo-7H-pyrido[1,2,3-de][1,4]benzoxazin-6-carbonsäure-Derivate gemäß Ansprüchen 1 bis 3, zur Bekämpfung von Virusinfektionen.

**7.** 9-Fluor-7-oxo-7H-pyrido[1,2,3-de][1,4]benzoxazin-6-carbonsäure-Derivate gemäß Ansprüchen 1 bis 3, zur Bekämpfung der Hepatitis.

**8.** Arzneimittel enthaltend 9-Fluor-7-oxo-7H-pyrido[1,2,3-de][1,4]benzoxazin-6-carbonsäure-Derivate gemäß Ansprüchen 1 bis 3.

**9.** Antivirale Mittel enthaltend 9-Fluor-7-oxo-7H-pyrido[1,2,3de][1,4]benzoxazin-6-carbonsäure-Derivate gemäß Ansprüchen 1 bis 3.

**10.** Verwendung von 9-Fluor-7-oxo-7H-pyrido[1,2,3-de][1,4]benzoxazin-6-carbonsäure-Derivate gemäß Ansprüchen 1 bis 3 bei der Herstellung von Arzneimitteln.

Europäisches Patentamt

# EUROPÄISCHER RECHERCHENBERICHT

Nummer der Anmeldung

EP 93 10 4662

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 253 235 (BAYER AG)<br>* Ansprüche 1,8 *<br>--- | 1,8 | C07D498/06<br>A61K31/535<br>//(C07D498/06, 265:00,221:00) |
| D,X | JOURNAL OF HETEROCYCLIC CHEMISTRY.<br>Bd. 28, Nr. 4, 1991, PROVO US<br>Seiten 1067 - 1074<br>TETSUO OKADA ET AL 'Synthesis and antibacterial activities of novel oxazine and thiazine ring-fused tricyclic quinolonecarboxylic acids: 10-(alicyclic amino)-9-fluoro-7-oxo-7H-pyrido(1,2,3-de)(1,4)benzoxazine-6-carboxylic acids and the corresponding 1-thia congeners'<br>* das ganze Dokument *<br>----- | 1,8 | |

RECHERCHIERTE SACHGEBIETE (Int. Cl.5 )

C07D
A61K

Der vorliegende Recherchenbericht wurde für alle Patentansprüche erstellt

| Recherchenort | Abschlußdatum der Recherche | Prüfer |
|---|---|---|
| DEN HAAG | 07 JULI 1993 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)